# EUROPÄISCHE PATENTSCHRIFT

(11) **EP 0 589 331 B1**
(45) Veröffentlichungstag und Bekanntmachung des Hinweises auf die Patenterteilung: **09.04.1997**
(21) Anmeldenummer: 93114723.5
(22) Anmeldetag: 14.09.1993
(51) Int. Cl.: C07C 25/24, C09K 19/18, C09K 19/30, C09K 19/34, C07C 22/08, C07C 43/225, C07D 213/24, C07D 239/26, C07D 319/06, C07D 405/06

(54) **Acetylen Derivate und sie enthaltende flüssigkristalline Mischungen**
Acetylene derivatives and liquid crystalline mixtures containing them
Dérivés de l'acétylène et mélanges de cristaux liquides les contenant

(30) Priorität: 25.09.1992 CH 3006/92
(43) Veröffentlichungstag der Anmeldung: 30.03.1994
(73) Patentinhaber: MERCK PATENT GmbH, 64293 Darmstadt (DE)
(72) Erfinder: Schadt, Martin, CH-4411 Seltisberg (CH); Villiger, Alois, CH-4056 Basel (CH)

(56) Entgegenhaltungen:
- EP-A- 0 409 634
- WO-A-88/07523
- GB-A- 2 155 465
- CHEMICAL ABSTRACTS, vol. 95, no. 25, 21. Dezember 1981, Columbus, Ohio, US; abstract no. 219871, & ADV. LIQ. CRYST. RES. APPL., PROC. LIQ. CRYST. CONF. SOC. COUNTRIES, 3RD 1979 (PUB.1981) Bd. 2 Seiten 1029 - 38 ADOMENAS, P. 'Acetylenic liquid crystals available by Castro reaction'

## Beschreibung

Die vorliegende Erfindung betrifft flüssigkristalline Acetylen-Derivate, flüssigkristalline Mischungen, die solche Verbindungen enthalten, sowie deren Verwendung für elektro-optische Zwecke.

Flüssigkristalline Acetylen-Derivaten sind schon aus WO 88/07523 bekannt.

Da die optischen Eigenschaften von flüssigen Kristallen durch eine angelegte Spannung beeinflusst werden können, werden solche Substanzen vor allem als Dielektrika in Anzeigevorrichtungen eingesetzt. Elektro-optische Vorrichtungen auf der Basis von Flüssigkristallen sind dem Fachmann bestens bekannt und können auf verschiedenen Effekten beruhen. Die gebräuchlichsten Anzeigevorrichtungen beruhen auf dem Schadt-Helfrich-Effekt und besitzen eine verdrillt nematische Struktur. Beispiele solcher Zellen sind die TN-Zelle ("twisted nematic") und die STN-Zelle (supertwisted nematic).

Die Flüssigkristallmaterialien für solche Zellen müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Sie sollen bei üblichen Betriebstemperaturen eine geeignete Mesophase besitzen; beispielsweise für die oben genannten Zellen eine nematische oder cholesterische Mesophase. Weiter sollte die dielektrische Anisotropie möglichst hoch sein.

Da Flüssigkristalle in der Regel als Mischungen mehrerer Komponenten zur Anwendung gelangen, ist es auch wichtig, dass die Komponenten untereinander gut mischbar sind.

Mit der vorliegenden Erfindung werden flüssigkristalline Verbindungen, die den obengenannten Anforderungen entsprechen, zur Verfügung gestellt.

Gegenstand der vorliegenden Erfindung sind Verbindungen der allgemeinen Formel: 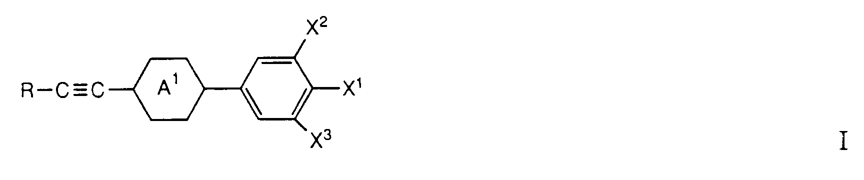 worin
- R: eine Alkylgruppe mit 1 bis 12 Kohlenstoffatomen oder eine Gruppe der Formel 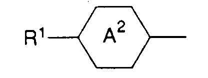 bedeutet;
- Ring A¹: unsubstituiertes oder mit Fluor substituiertes 1,4-Phenylen, Pyridin-2,5-diyl oder Pyrimidin-2,5-diyl darstellt;
- Ring A²: trans-1,4-Cyclohexylen oder trans-1,3-Dioxan-2,5-diyl bedeutet;
- R¹: eine Alkyl-, Alkenyl-, oder Alkoxyalkylgruppe mit 1 bis 12, bzw. 2 bis 12 Kohlenstoffatomen darstellt;
- X¹: Fluor, Chlor, Brom, Trifluormethyl, Trifluormethoxy oder Difluormethoxy bedeutet; und
- X², X³: Wasserstoff oder Fluor darstellen.

Die erfindungsgemässen Verbindungen zeichnen sich durch eine hohe optische Anisotropie aus. Durch die polaren Substituenten am Phenylring wird ihre dielektrische Anisotropie erhöht. Ferner besitzen diese Verbindungen eine erstaunlich niedrige Viskosität und einen breiten, günstigen Mesophasenbereich, sowie einen vergleichsweise hohen Klärpunkt. All diese Eigenschaften bewirken, dass Mischungen enthaltend Verbindungen der allgemeinen Formel I, sehr gute Mesophasen aufweisen und zudem niedrige Schwellenspannung, kurze Ansprechzeiten und einen hohen Kontrast haben. Die Verbindungen der Formel I eignen sich daher insbesondere für die Anwendung in TN- und STN-Zellen.

Der Ausdruck "Alkyl mit 1 bis 12 Kohlenstoffatomen" umfasst geradkettige und verzweigte (gegebenenfalls chirale) Alkylreste wie beispielsweise Methyl, Aethyl, Propyl, Butyl, Pentyl, Hexyl, Heptyl, Octyl, Nonyl, Decyl, Undecyl, Dodecyl, 1-Methylpropyl, 1-Methylbutyl, 1-Methylpentyl, 1-Methylhexyl, 1-Methylheptyl, 2-Methylbutyl, 3-Methylpentyl und dergleichen. Besonders bevorzugt sind die geradkettigen Alkylreste mit 1 bis 7, insbesondere jedoch diejenigen mit 2 bis 5 Kohlenstoffatomen.

Der Ausdruck "Alkenyl mit 2 bis 12 Kohlenstoffatomen" umfasst 1E-Alkenyl, 3E-Alkenyl, 4-Alkenyl (wobei E die Konfiguration der Doppelbindung angibt) oder Alkenyl mit endständiger Doppelbindung und dergleichen. Beispiele solcher Gruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl (wobei Z die Konfiguration der Doppelbindung angibt), 5-Hexenyl, 6-Heptenyl, 7-Octenyl und dergleichen. Besonders bevorzugt sind die geradkettigen Alkenylreste mit 2 bis 7, insbesondere jedoch diejenigen mit 2 bis 5 Kohlenstoffatomen.

Der Ausdruck "Alkyloxyalkyl mit 2 bis 12 Kohlenstoffatomen" umfasst Gruppen wie beispielsweise Methoxymethyl, Methoxyäthyl, Methoxypropyl, Methoxybutyl, Aethoxymethyl, Aethoxyäthyl, Aethoxypropyl, Aethoxybutyl und dergleichen. Bevorzugte Alkyloxyalkyl-Reste haben 2 bis 7 Kohlenstoffatome, insbesondere 2 bis 5 Kohlenstoffatome.

Der Ausdruck "unsubstitutiertes oder mit Fluor substituiertes 1,4-Phenylen" umfasst 1,4-Phenylen, 2-Fluor-1,4-phenylen und dergleichen.

Bevorzugte Verbindungen der Formel I sind die bicyclische Verbindungen der Formeln: 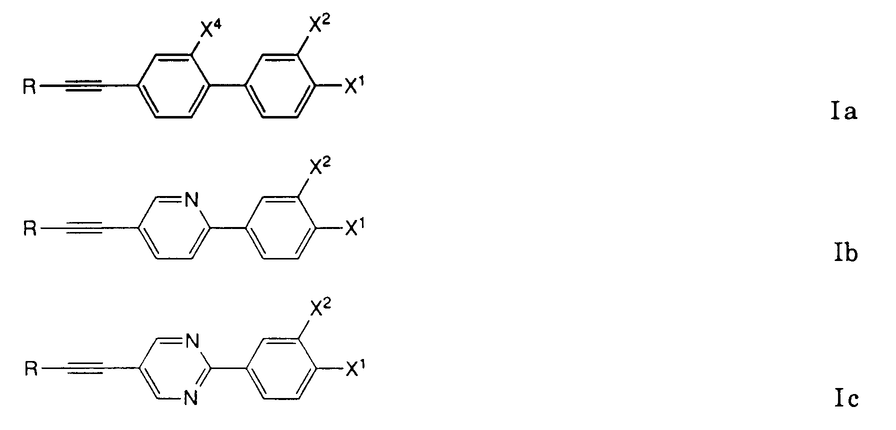 worin R eine Alkylgruppe mit 1 bis 12 Kohlenstoffatomen bedeutet; X¹ und X² die obengenannten Bedeutungen haben, und X⁴ Wasserstoff oder Fluor bedeutet,
sowie die tricyclischen Verbindungen der Formeln: 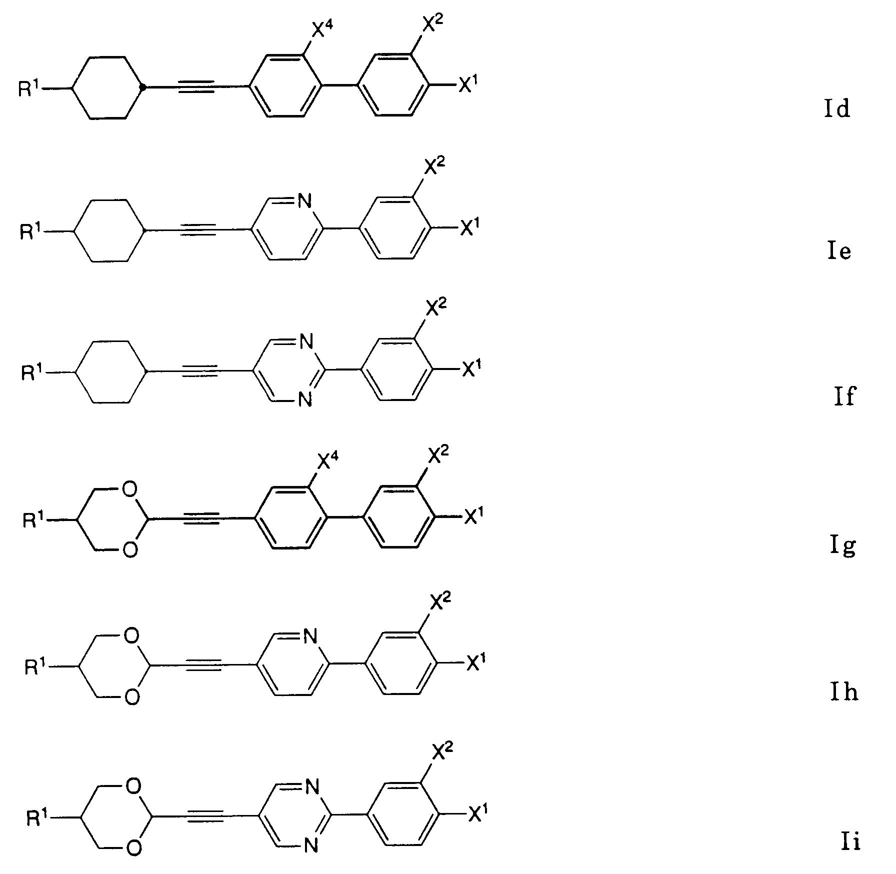 worin R¹, X¹, X² und X⁴ die obengenannten Bedeutungen haben.

Die Verbindungen der Formeln Ia-Ii weisen eine ausgeprägte, breite nematische Mesophase mit hohem Klärpunkt auf. Besonders bevorzugt sind die Verbindungen der Formeln Id-Ii, insbesondere diejenigen der Formeln Id-If.

In den Verbindungen der Formeln Ia, Id und Ig steht X⁴ vorzugsweise für Wasserstoff.

Die Verbindungen der allgemeinen Formel I, worin R Alkyl oder trans-4-Alkylcyclohexyl bedeutet, können auf an sich bekannte Weise z.B. nach dem in Schema 1 aufgezeichneten Weg hergestellt werden, d.h. durch Kupplung eines geeigneten Acetylen-Derivates II mit der entsprechenden Bromverbindung III in Gegenwart von Tetrakis(triphenylphosphin)palladium und CuBr. Die Reaktion erfolgt vorzugsweise in Triäthylamin.

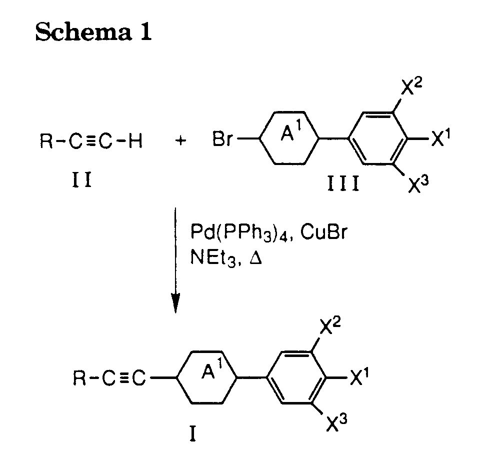

Die zur Kupplung mit den Acetylen-Derivaten verwendeten Bromverbindungen können in an sich bekannter Weise zum Beispiel nach dem in Schema 2 angegebenen Weg hergestellt werden.

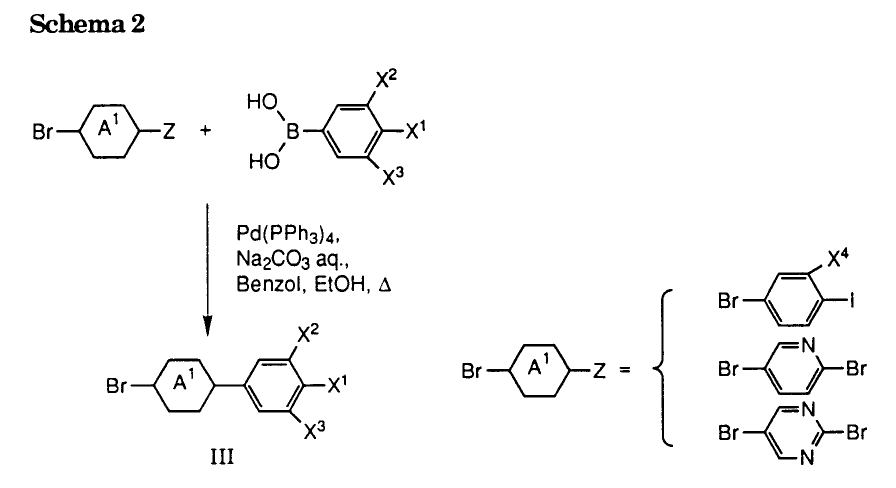

Die tricyclischen Cyclohexylen-Derivate der Formeln Id-If, worin R¹ Alkenyl oder Alkoxyalkyl bedeutet, können zum Beispiel gemäss Schema 3, d.h. durch Kupplung eines Dioxaspiro[4,5]decan-Acetylenderivates IV mit der entsprechenden Bromverbindung III hergestellt werden;

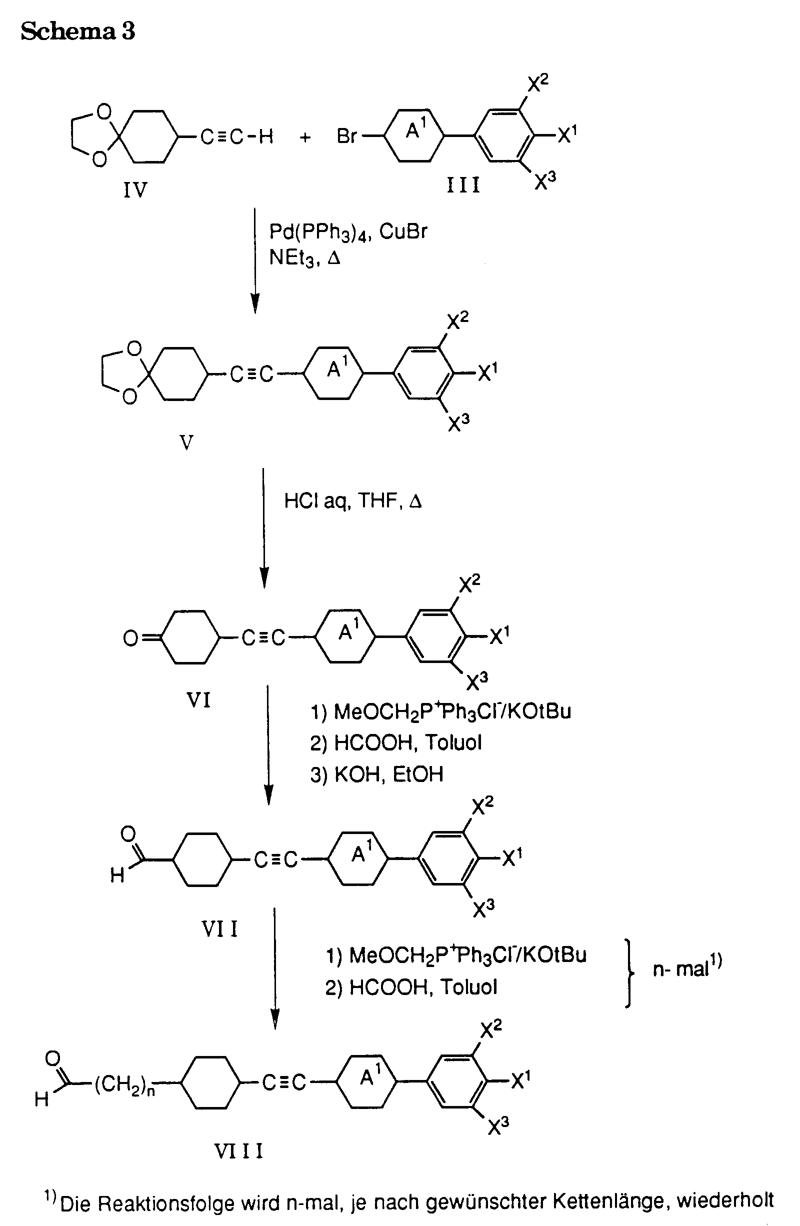

Die Seitenkette R¹ kann dabei durch wiederholte Homologisierung nach Wittig aufgebaut werden (siehe auch Schema 5).

Die tricyclischen Dioxan-Derivate der Formeln Ig-Ii können beispielsweise gemäss Schema 4, d.h. durch Kupplung eines geeigneten Acetal-Acetylen-Derivates IX und der entsprechenden Bromverbindung III und anschliessender Umacetalisierung der Verbindung X hergestellt werden:

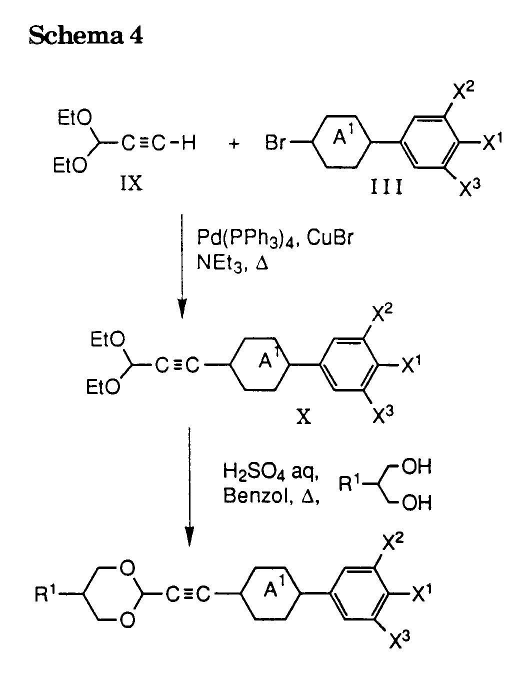

In den Verbindungen der Formel I, worin R¹ Alkenyl bedeutet, kann die Doppelbindung auf an sich bekannte Weise gemäss, z.B. Schema 5, aus den entsprechenden Aldehyden VIII durch eine Wittigreaktion und anschliessende Isomerisierung eingeführt werden.

In den Verbindungen der Formel I, worin R¹ Alkyloxyalkyl bedeutet, kann, z.B. ebenfalls gemäss Schema 5 dieser Rest durch Hydrierung des entsprechenden Aldehydes VIII und anschliessender Verätherung mit dem entsprechenden Alkohol aufgebaut werden.

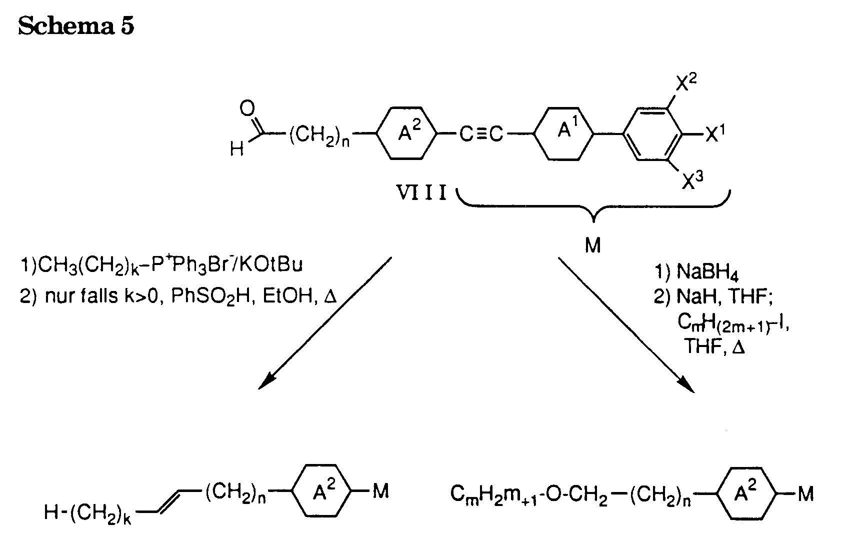

In den Schemata 1 bis 5 haben die Symbole R, R¹, A¹, X¹, X², X³ und X⁴ die obengenannten Bedeutungen.

Die Verbindungen der Formel I können in Form von Gemischen untereinander und/oder mit anderen Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft daher ebenfalls flüssigkristalline Gemische mit mindestens 2 Komponenten, welche dadurch gekennzeichnet sind, dass mindestens eine Komponente eine Verbindung der Formel I ist.

Aufgrund der guten Löslichkeit der Verbindungen der Formel I in anderen Flüssigkristallmaterialien, und aufgrund ihrer guten Mischbarkeit untereinander, kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-30 Gew.% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln: 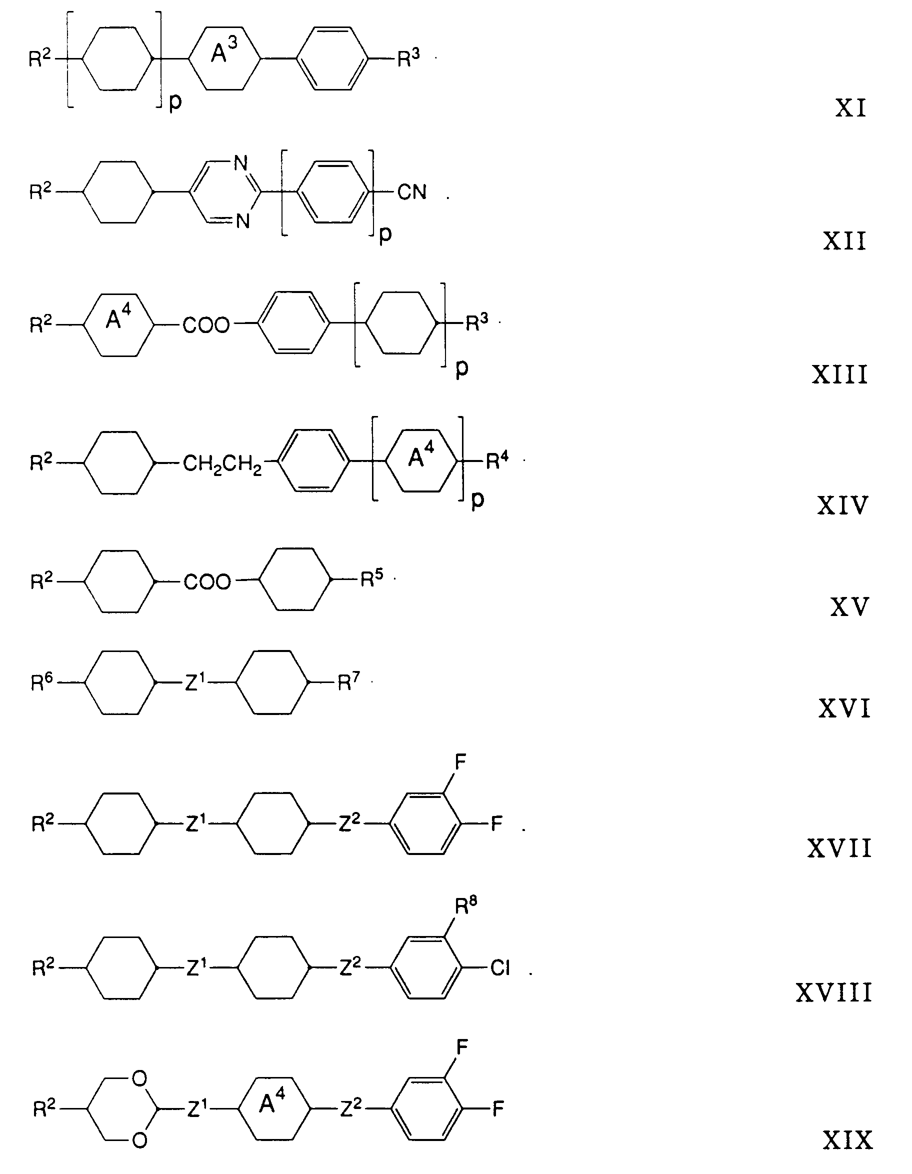 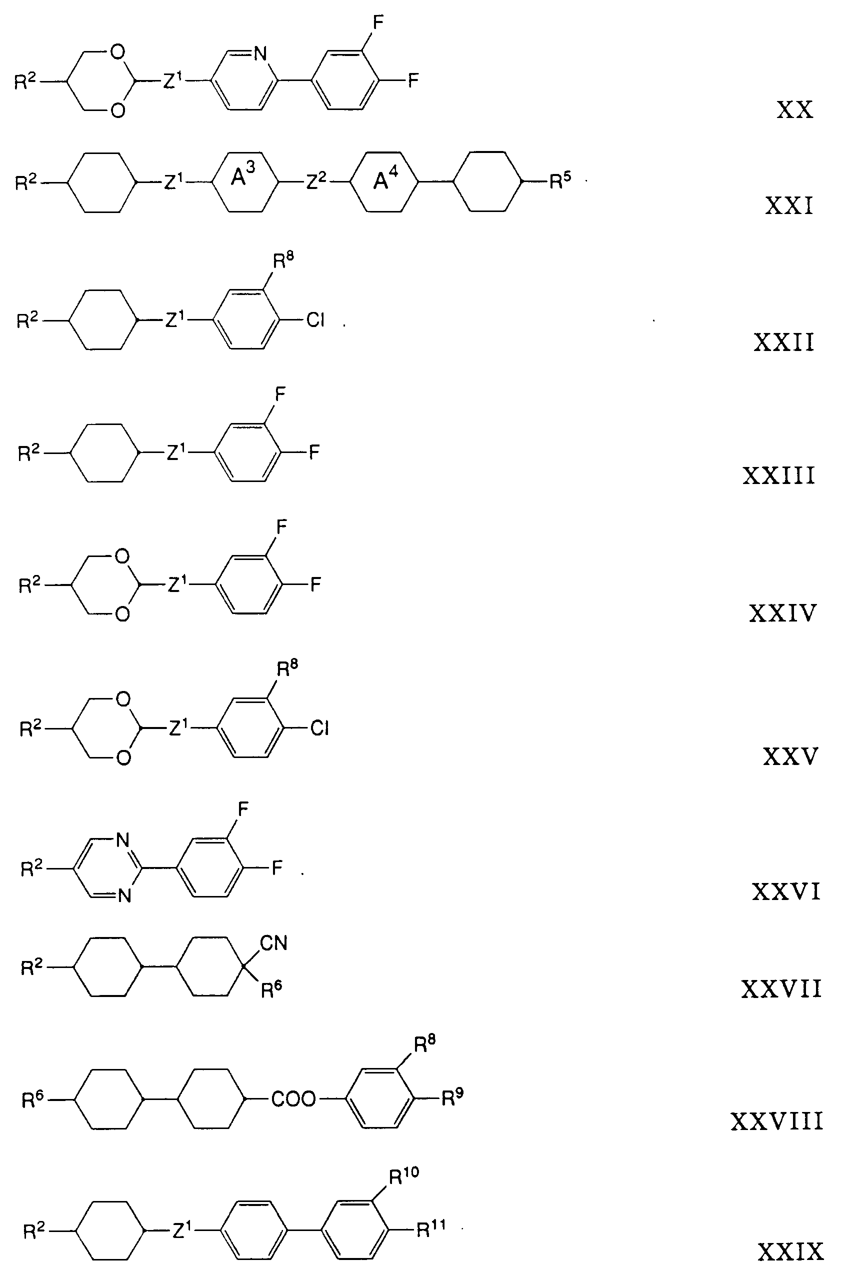 worin
- R²,R⁵: Alkyl, Alkoxyalkyl, 3E-Alkenyl, 4-Alkenyl oder an gesättigten Ringen auch 1E-Alkenyl bedeutet;
- p: 0 oder 1 bedeutet;
- Ring A³: 1,4-Phenylen, Pyridin-2,5-diyl, Pyrimidin-2,5-diyl, trans-1,4-Cyclohexylen oder trans-1,3-Dioxan-2,5-diyl bezeichnet;
- R³: Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt;
- Ring A⁴: 1,4-Phenylen oder trans-1,4-Cyclohexylen bedeutet;
- R⁴: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet;
- R⁶: Alkyl, 1E-Alkenyl, 3E-Alkenyl oder 4-Alkenyl bedeutet;
- R⁷: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt;
- Z¹, Z²: eine einfache Kovalenzbindung oder -CH₂CH₂- bezeichnen, wobei zwei aromatische Ringe immer durch eine einfache Kovalenzbindung verknüpft sind;
- R⁸: Wasserstoff, Fluor oder Chlor bedeutet;
- R⁹: Cyano, Fluor oder Chlor darstellt;
- R¹⁰: Wasserstoff oder Fluor bezeichnet; und
- R¹¹: Fluor oder Chlor darstellt.

Der obenerwähnte Ausdruck "aromatische Ringe" bezeichnet in diesem Zusammenhang Ringe wie beispielsweise 1,4-Phenylen, Pyridin-2,5-diyl oder Pyrimidin-2,5-diyl. Der Ausdruck "gesättigte Ringe" bezeichnet trans-1,4-Cyclohexylen oder 1,3-Dioxan-2,5-diyl.

Reste R² bis R⁷ besitzen vorzugsweise je 1 bis 12 Kohlenstoffatome, besonders bevorzugt je 1 bis 7 Kohlenstoffatome. Gerad-kettige Reste sind im allgemeinen bevorzugt. Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen wie beispielsweise Methyl, Aethyl, Propyl, Butyl, Pentyl, Hexyl oder Heptyl.

Der Ausdruck "Alkyloxyalkyl" bedeutet in diesem Zusammenhang vorzugsweise geradkettige Reste wie beispielsweise Methoxymethyl, Aethoxymethyl, Propyloxymethyl, Butyloxymethyl und dergleichen.

Der Ausdruck "Alkyloxy" bedeutet in diesem Zusammenhang vorzugsweise geradkettige Reste wie beispielsweise Methoxy, Aethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl" bedeutet in diesem Zusammenhang vorzugsweise geradkettige Alkenylreste, in denen die Doppelbindung sich in 4-Stellung befindet, wie beispielsweise 4-Pentenyl, 4-Hexenyl, 4-Heptenyl und dergleichen.

Der Ausdruck "2E- bzw. 3Z-Alkenyloxy" bedeutet in diesem Zusammenhang vorzugsweise geradkettige Alkenyloxyreste, in denen die Doppelbindung sich in 2- bzw 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste, in denen die Dreifachbindung sich in 1-Stellung befindet, wie beispielsweise Aethinyl, 1-Propinyl, 1-Butinyl, 1-Pentinyl und dergleichen.

Die erfindungsgemässen Mischungen können gegebenenfalls weitere optisch aktive Verbindungen (z.B. optisch aktive 4'-Alkyl. oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn bezeichnet die optische Anisotropie.

Die nachstehenden Beispiele sollen die Eigenschaften der erfindungsgemässen Verbindungen weiter veranschaulichen.

### Beispiel 1

Ein Gemisch aus 1,506 g 4-Brom-4'-fluorbiphenyl, 1,127 g (trans-4-Propylcyclohexyl)acetylen, 0,138 g Tetrakis(triphenylphosphin)palladium, 0,052 g Kupfer(I)bromid und 36 ml Triäthylamin wurde 4,5 Stunden zum Sieden erhitzt. Das Reaktionsgemisch wurde nach dem Erkalten mit 100 ml Diäthyläther verdünnt, mit zweimal je 40 ml Wasser gewaschen, über Natriumsulfat getrocknet, filtriert und eingedampft. Das rohe 4-[(trans-4-Propylcyclohexyl)äthinyl]-4'-fluorbiphenyl wurde durch Chromatographie an 12 g Kieselgel mit Hexan/Aethylacetat (Vol. 49:1) und zweimalige Umkristallisation aus Hexan gereinigt; Smp. (C-N) 110,6°C, Klp. (N-I) 179°C.

In analoger Weise können hergestellt werden:
4-[(trans-4-Aethylcyclohexyl)äthinyl]-4'-fluorbiphenyl;
4-[(trans-4-Butylcyclohexyl)äthinyl]-4'-fluorbiphenyl, Smp. (C-S_{A}) 88,7°C, S_{A}-N 94,1°C, Klp. (N-I) 171°C;
4-[(trans-4-Pentylcyclohexyl)äthinyl]-4'-fluorbiphenyl;
4-[(trans-4-Hexylcyclohexyl)äthinyl]-4'-fluorbiphenyl;
4-[(trans-4-Heptylcyclohexyl)äthinyl]-4'-fluorbiphenyl;
4-[(trans-4-Propylcyclohexyl)äthinyl]-3',4'-difluorbiphenyl, Smp. (C-N) 80,3°C, Klp. (N-I) 132,5°C;
4-[(trans-4-Butylcyclohexyl)äthinyl]-3',4'-difluorbiphenyl, Smp. (C-N) 65,8°C, S_{A}-N 47,4°C, Klp. (N-I) 127°C;
4-[(trans-4-Pentylcyclohexyl)äthinyl]-3',4'-difluorbiphenyl;
4-[(trans-4-Propylcyclohexyl)äthinyl]-4'-chlorbiphenyl, Smp. (C-N) 168,7°C, Klp. (N-I) 208°C;
4-[(trans-4-Butylcyclohexyl)äthinyl]-4'-chlorbiphenyl, Smp. (C-S_{A}) 138,7°C, S_{A}-N 156,7°C, Klp. (N-I) 200°C;
4-[(trans-4-Pentylcyclohexyl)äthinyl]-4'-chlorbiphenyl;
4-[(trans-4-Propylcyclohexyl)äthinyl]-4'-chlor-3'-fluorbiphenyl, Smp. (C-N) 124,4°C, Klp. (N-I) 159,5°C;
4-[(trans-4-Butylcyclohexyl)äthinyl]-4'-chlor-3'-fluorbiphenyl, Smp. (C-S_{A}) 88,5°C, S_{A}-N 95,6°C, Klp. (N-I) 154°C;
4-[(trans-4-Pentylcyclohexyl)äthinyl]-4'-chlor-3'-fluorbiphenyl;
4-[(trans-4-Propylcyclohexyl)äthinyl]-4'-brombiphenyl;
4-[(trans-4-Butylcyclohexyl)äthinyl]-4'-brombiphenyl;
4-[(trans-4-Propylcyclohexyl)äthinyl]-4'-trifluormethoxybiphenyl;
4-[(trans-4-Butylcyclohexyl)äthinyl]-4'-trifluormethoxybiphenyl;
4-[(trans-4-Propylcyclohexyl)äthinyl]-4'-difluormethoxybiphenyl;
4-[(trans-4-Butylcyclohexyl)äthinyl]-4'-difluormethoxybiphenyl;
4-[(trans-4-Propylcyclohexyl)äthinyl]-4'-difluormethoxy-3'-fluorbiphenyl;
4-[(trans-4-Butylcyclohexyl)äthinyl]-4'-difluormethoxy-3'-fluorbiphenyl;
4-[(trans-4-Butylcyclohexyl)äthinyl]-3',4',5'-trifluorbiphenyl;
4-[(trans-4-Propylcyclohexyl)äthinyl]-4'-(difluoromethoxy)-3',5'-difluorbiphenyl, Smp. (C-N) 62,2°C, Klp. (N-I) 110°C.
4-[(trans-4-Butylcyclohexyl)äthinyl]-3',5'-difluor-4'-chlorbiphenyl;
4-(1-Butinyl)-4'-fluorbiphenyl;
4-(1-Pentinyl)-4'-fluorbiphenyl;
4-(1-Hexinyl)-4'-fluorbiphenyl, Klp. (S-I) 73,7°C;
4-(1-Heptinyl)-4'-fluorbiphenyl;
4-(1-Pentinyl)-3',4'-difluorbiphenyl;
4-(1-Hexinyl)-3',4'-difluorbiphenyl, Smp. (C-S) 18.8°C, Klp. (S-I) 22,2°C;
4-(1-Heptinyl)-3',4'-difluorbiphenyl, Smp. (C-I) 20,6°C, Klp. (S-I) 2°C;
4-(1-Butinyl)-4'-chlorbiphenyl;
4-(1-Pentinyl)-4'-chlorbiphenyl;
4-(1-Hexinyl)-4'-chlorbiphenyl;
4-(1-Heptinyl)-4'-chlorbiphenyl;
4-(1-Butinyl)-4'-chlor-3'-fluorbiphenyl;
4-(1-Pentinyl)-4'-chlor-3'-fluorbiphenyl;
4-(1-Hexinyl)-4'-chlor-3'-fluorbiphenyl;
4-(1-Heptinyl)-4'-chlor-3'-fluorbiphenyl;
4-(1-Hexinyl)-4'-(trifluormethoxy)biphenyl;
4-(1-Hexinyl)-4'-(difluormethoxy)biphenyl;
4-(1-Hexinyl)-4'-(difluormethoxy)-3'-fluorbiphenyl;
4-(1-Pentinyl)-4'-(difluormethoxy)-3',5'-difluorphenyl, Smp. (C-I) 19,6°C;
4-(1-Hexinyl)-4'-(difluormethoxy)-3',5'-difluorphenyl, Smp. (C-I) 34,4°C;
4-(1-Heptinyl)-4'-(difluormethoxy)-3',5'-difluorphenyl, Smp. (C-I) 6,3°C;
4-(1-Hexinyl)-4'-(trifluormethyl)-3'-fluorbiphenyl;
5-[(trans-4-Aethylcyclohexyl)äthinyl]-2-(4-fluorphenyl)pyridin;
5-[(trans-4-Propylcyclohexyl)äthinyl]-2-(4-fluorphenyl)pyridin, Smp. (C-N) 73,7°C, S_{A}-N 72,0°C, Klp. (N-I) 178°C;
5-[(trans-4-Butylcyclohexyl)äthinyl]-2-(4-fluorphenyl)pyridin, Smp. (C-S_{A}) 66,0°C, S_{A}-N 87,5°C, Klp. (N-I) 170°C;
5-[(trans-4-Pentylcyclohexyl)äthinyl]-2-(4-fluorphenyl)pyridin;
5-[(trans-4-Hexylcyclohexyl)äthinyl]-2-(4-fluorphenyl)pyridin;
5-[(trans-4-Heptylcyclohexyl)äthinyl]-2-(4-fluorphenyl)pyridin;
5-[(trans-4-Propylcyclohexyl)äthinyl]-2-(3,4-difluorphenyl)pyridin, Smp. (C-N) 67,3°C, S_{A}-N 47,2°C, Klp. (N-I) 137,5°C;
5-[(trans-4-Butylcyclohexyl)äthinyl]-2-(3,4-difluorphenyl)pyridin, Smp. (C-N) 76,2°C, S_{A}-N 55,0°C, Klp. (N-I) 133°C;
5-[(trans-4-Pentylcyclohexyl)äthinyl]-2-(3,4-difluorphenyl)pyridin;
5-[(trans-4-Aethylcyclohexyl)äthinyl]-2-(4-chlorphenyl)pyridin;
5-[(trans-4-Propylcyclohexyl)äthinyl]-2-(4-chlorphenyl)pyridin, Smp. (C-S_{A}) 118,0°C, S_{A}-N 143,0°C, Klp. (N-I) 208°C;
5-[(trans-4-Butylcyclohexyl)äthinyl]-2-(4-chlorphenyl)pyridin, Smp. (C-S_{A}) 90,3°C, S_{A}-N 151,8°C, Klp. (N-I) 202°C;
5-[(trans-4-Pentylcyclohexyl)äthinyl]-2-(4-chlorphenyl)pyridin;
5-[(trans-4-Propylcyclohexyl)äthinyl]-2-(4-chlor-3-fluorphenyl)pyridin, Smp. (C-S_{A}) 86,6°C, S_{A}-N 94,3°C, Klp. (N-I) 165°C;
5-[(trans-4-Butylcyclohexyl)äthinyl]-2-(4-chlor-3-fluorphenyl)pyridin, Smp. (C-S_{A}) 66,1°C, S_{A}-N 101,5°C, Klp. (N-I) 160°C;
5-[(trans-4-Pentylcyclohexyl)äthinyl]-2-(4-chlor-3-fluorphenyl)pyridin;
5-[(trans-4-Butylcyclohexyl)äthinyl]-2-(4-bromphenyl)pyridin;
5-[(trans-4-Butylcyclohexyl)äthinyl]-2-[(4-(trifluormethoxy)phenyl]pyridin;
5-[(trans-4-Butylcyclohexyl)äthinyl]-2-[(4-(difluormethoxy)phenyl]pyridin;
5-[(trans-4-Butylcyclohexyl)äthinyl]-2-[(4-(trifluormethyl)phenyl]pyridin;
5-[(trans-4-Propylcyclohexyl)äthinyl]-2-(3,4,5-fluorphenyl)pyridin;
5-[(trans-4-Butylcyclohexyl)äthinyl]-2-(4-chlor-3,5-difluorphenyl)pyridin;
5-(1-Butinyl)-2-(4-fluorphenyl)pyridin;
5-(1-Pentinyl)-2-(4-fluorphenyl)pyridin, Smp (C-I) 58,3°C, Klp. (S_{A}-I) 48,8°C;
5-(1-Hexinyl)-2-(4-fluorphenyl)pyridin, Smp (C-S_{A}) 20,7°C, Klp. (S_{A}-I) 37,0°C;
5-(1-Heptinyl)-2-(4-fluorphenyl)pyridin, Smp (C-I) 25,2°C, Klp. (S_{A}-I) 23,9°C;
5-(1-Pentinyl)-2-(3,4-difluorphenyl)pyridin;
5-(1-Hexinyl)-2-(3,4-difluorphenyl)pyridin, Smp. (C-I) 50,4°C, Klp. (S_{A}-I) 25°C;
5-(1-Heptinyl)-2-(3,4-difluorphenyl)pyridin;
5-(1-Pentinyl)-2-(4-chlorphenyl)pyridin;
5-(1-Hexinyl)-2-(4-chlorphenyl)pyridin, Smp. (C-S_{A}) 26°C, Klp. (S_{A}-I) 87,5°C;
5-(1-Heptinyl)-2-(4-chlorphenyl)pyridin;
5-(1-Pentinyl)-2-(4-chlor-3-fluorphenyl)pyridin, Smp. (C-I) 63,3°C, Klp. (S_{A}-I) 61,9°C;
5-(1-Hexinyl)-2-(4-chlor-3-fluorphenyl)pyridin;
5-(1-Heptinyl)-2-(4-chlor-3-fluorphenyl)pyridin;
5-(1-Hexinyl)-2-(4-bromphenyl)pyridin;
5-(1-Hexinyl)-2-[4-(trifluormethoxy)phenyl]pyridin;
5-(1-Hexinyl)-2-[4-(difluormethoxy)phenyl]pyridin;
5-(1-Hexinyl)-2-[4-(difluormethoxy)-3-fluorphenyl]pyridin;
5-(1-Hexinyl)-2-[4-(trifluormethyl)phenyl]pyridin;
5-[(trans-4-Aethylcyclohexyl)äthinyl]-2-(4-fluorphenyl)pyrimidin;
5-[(trans-4-Propylcyclohexyl)äthinyl]-2-(4-fluorphenyl)pyrimidin, Smp. (C-N) 117,3°C, S_{A}-N 95,8°C, Klp. (N-I) 180,5°C;
5-[(trans-4-Butylcyclohexyl)äthinyl]-2-(4-fluorphenyl)pyrimidin, Smp. (C-S_{A}) 104,9°C, S_{A}-N 109,5°C, Klp. (N-I) 175°C;
5-[(trans-4-Pentylcyclohexyl)äthinyl]-2-(4-fluorphenyl)pyrimidin;
5-[(trans-4-Hexylcyclohexyl)äthinyl]-2-(4-fluorphenyl)pyrimidin;
5-[(trans-4-Heptylcyclohexyl)äthinyl]-2-(4-fluorphenyl)pyrimidin;
5-[(trans-4-Propylcyclohexyl)äthinyl]-2-(3,4-difluorphenyl)pyrimidin, Smp. (C-N) 109,3°C, Klp. (N-I) 152°C;
5-[(trans-4-Butylcyclohexyl)äthinyl]-2-(3,4-difluorphenyl)pyrimidin, Smp. (C-N) 99,6°C, S_{A}-N 97,3°C, Klp. (N-I) 147,5°C;
5-[(trans-4-Pentylcyclohexyl)äthinyl]-2-(3,4-difluorphenyl)pyrimidin;
5-[(trans-4-Aethylcyclohexyl)äthinyl]-2-(4-chlorphenyl)pyrimidin;
5-[(trans-4-Propylcyclohexyl)äthinyl]-2-(4-chlorphenyl)pyrimidin, Smp. (C-S_{A}) 131,1°C, S_{A}-N 148,0°C, Klp. (N-I) 208°C;
5-[(trans-4-Butylcyclohexyl)äthinyl]-2-(4-chlorphenyl)pyrimidin, Smp. (C-S_{A}) 99,5°C, S_{A}-N 156°C, Klp. (N-I) 202,5°C;
5-[(trans-4-Pentylcyclohexyl)äthinyl]-2-(4-chlorphenyl)pyrimidin;
5-[(trans-4-Propylcyclohexyl)äthinyl]-2-(4-chlor-3-fluorphenyl)pyrimidin, Smp. (C-S_{A}) 96,1°C, S_{A}-N 120,6°C, Klp. (N-I) 171,5°C;
5-[(trans-4-Butylcyclohexyl)äthinyl]-2-(4-chlor-3-fluorphenyl)pyrimidin, Smp. (C-S_{A}) 111,3°C, S_{A}-N 129,3°C, Klp. (N-I) 171°C;
5-[(trans-4-Pentylcyclohexyl)äthinyl]-2-(4-chlor-3-fluorphenyl)pyrimidin;
5-(1-Pentinyl)-2-(4-fluorphenyl)pyrimidin;
5-(1-Hexinyl)-2-(4-fluorphenyl)pyrimidin, Smp. (C-I) 69,5°C, Klp. (S_{A}-I) 41,0°C;
5-(1-Heptinyl)-2-(4-fluorphenyl)pyrimidin;
5-(1-Butinyl)-2-(3,4-difluorphenyl)pyrimidin;
5-(1-Pentinyl)-2-(3,4-difluorphenyl)pyrimidin, Smp. (C-I) 73,1°C, Klp. (S_{A}-I) 62,6°C;
5-(1-Hexinyl)-2-(3,4-difluorphenyl)pyrimidin, Smp. (C-S_{A}) 55,5°C, Klp. (S_{A}-I) 61,5°C;
5-(1-Heptinyl)-2-(3,4-difluorphenyl)pyrimidin;
5-(1-Octinyl)-2-(3,4-difluorphenyl)pyrimidin;
5-(1-Noninyl)-2-(3,4-difluorphenyl)pyrimidin;
5-(1-Pentinyl)-2-(4-chlorphenyl)pyrimidin, Smp. (C-I) 121,2°C;
5-(1-Hexinyl)-2-(4-chlorphenyl)pyrimidin, Smp. (C-S_{A}) 76,3°C, Klp. (S_{A}-I) 82,6°C;
5-(1-Heptinyl)-2-(4-chlorphenyl)pyrimidin, Smp. (C-S_{A}) 79,8°C, Klp. (S_{A}-I) 80,8°C;
5-(1-Pentinyl)-2-(4-chlor-3-fluorphenyl)pyrimidin, Smp. (C-I) 101,4°C, Klp. (S_{A}-I) 88,7°C;
5-(1-Hexinyl)-2-(4-chlor-3-fluorphenyl)pyrimidin, Smp. (C-I) 81,1°C, Klp. (S_{A}-I) 78,4°C;
5-(1-Heptinyl)-2-(4-chlor-3-fluorphenyl)pyrimidin, Smp. (C-S_{A}) 72,1°C, Klp. (S_{A}-I) 72,6°C;
5-(1-Octinyl)-2-(4-chlor-3-fluorphenyl)pyrimidin;
5-(1-Hexinyl)-2-(4-bromphenyl)pyrimidin;
5-(1-Hexinyl)-2-(4-brom-3-fluorphenyl)pyrimidin;
5-(1-Hexinyl)-2-[4-(trifluormethoxy)phenyl]pyrimidin;
5-(1-Hexinyl)-2-[4-(difluormethoxy)phenyl]pyrimidin;
5-(1-Hexinyl)-2-[4-(difluormethoxy)-3-fluorphenyl]pyrimidin;
5-(1-Hexinyl)-2-[4-(trifluormethyl)phenyl]pyrimidin;
5-(1-Hexinyl)-2-[4-(trifluormethyl)-3-fluorphenyl]pyrimidin.

### Beispiel 2

a) Ein Gemisch aus 2,00 g rohem 5-Brom-2-(3,4-difluorphenyl)pyridin (GC-Reinheit 81%), 1,12 ml 3,3-Diäthoxy-1-propin, 0,138 g Tetrakis(triphenylphosphin)palladium, 0,052 g Kupfer(I)bromid und 36 ml Triäthylamin wurde 2 Stunden zum Sieden erhitzt. Das Reaktionsgemisch wurde nach dem Erkalten mit 100 ml Diäthyläther verdünnt, zweimal mit je 40 ml Wasser gewaschen, über Natriumsulfat getrocknet, filtriert, das Filtrat mit Aktivkohle kurz gekocht, filtriert und eingeengt. Das als braunschwarzes Oel erhaltene, rohe 5-(3,3-Diäthoxy-1-propinyl)-2-(3,4-difluorphenyl)pyridin, (2,58 g GC-Reinheit 72%) wurde direkt in der nächsten Stufe eingesetzt.
b) Ein Gemisch aus 0,86 g rohem 5-(3,3-Diäthoxy-1-propinyl)-2-(3,4-difluorphenyl)pyridin, 0,60 g 2-Propyl-1,3-propandiol, 50 ml Benzol, 24 Tropfen Wasser und 12 Tropfen 10%ige Schwefelsäure (v/v) wurde 0,5 Stunden bei 73°C gerührt, dann 1 Stunde zum Sieden erhitzt, wobei feuchtes Benzol abdestilliert und durch frisches ersetzt wurde. Das Reaktionsgemisch wurde mit je 30 ml gesättigter Natriumhydrogencarbonat-Lösung und Wasser gewaschen, über Natriumsulfat getrocknet, filtriert und eingeengt. Das rohe 5-[(trans-5-Propyl-1,3-dioxan-2-yl)äthinyl]-2-(3,4-difluorphenyl)pyridin wurde durch Chromatographie an 16 g Kieselgel mit Hexan/Aethylacetat (Vol. 9:1) und zweimalige Umkristallisation aus Aethylacetat gereinigt; Smp. (C-N) 124,7°C, Klp. (N-I) 128,4°C.

In analoger Weise können hergestellt werden:
5-[(trans-5-Propyl-1,3-dioxan-2-yl)äthinyl]-2-(4-fluorphenyl)pyridin;
5-[(trans-5-Butyl-1,3-dioxan-2-yl)äthinyl]-2-(4-fluorphenyl)pyridin;
5-[(trans-5-Pentyl-1,3-dioxan-2-yl)äthinyl]-2-(4-fluorphenyl)pyridin;
5-[(trans-5-(1E-Propenyl)-1,3-dioxan-2-yl)äthinyl]-2-(4-fluorphenyl)pyridin;
5-[(trans-5-(1E-Butenyl)-1,3-dioxan-2-yl)äthinyl]-2-(4-fluorphenyl)pyridin;
5-[(trans-5-(1E-Pentenyl)-1,3-dioxan-2-yl)äthinyl]-2-(4-fluorphenyl)pyridin;
5-[(trans-5-(3-Butenyl)-1,3-dioxan-2-yl)äthinyl]-2-(4-fluorphenyl)pyridin;
5-[(trans-5-(4-Pentenyl)-1,3-dioxan-2-yl)äthinyl]-2-(4-fluorphenyl)pyridin;
5-[(trans-5-Butyl-1,3-dioxan-2-yl)äthinyl]-2-(3,4-difluorphenyl)pyridin;
5-[(trans-5-Pentyl-1,3-dioxan-2-yl)äthinyl]-2-(3,4-difluorphenyl)pyridin;
5-[(trans-5-(1E-Propenyl)-1,3-dioxan-2-yl)äthinyl]-2-(3,4-difluorphenyl)pyridin, Smp. (C-N) 155,5°C, Klp. (N-I) 173°C;
5-[(trans-5-(1E-Butenyl)-1,3-dioxan-2-yl)äthinyl]-2-(3,4-difluorphenyl)pyridin;
5-[(trans-5-(1E-Pentenyl)-1,3-dioxan-2-yl)äthinyl]-2-(3,4-difluorphenyl)pyridin;
5-[(trans-5-(3-Butenyl-1,3-dioxan-2-yl)äthinyl]-2-(3,4-difluorphenyl)pyridin, Smp. (C-N) 109,2°C, S_{A}-N 91,5°C, Klp. (N-I) 118°C;
5-[(trans-5-(4-Pentenyl)-1,3-dioxan-2-yl)äthinyl]-2-(3,4-difluorphenyl)pyridin;
5-[(trans-5-(4-Propyl-1,3-dioxan-2-yl)äthinyl]-2-(4-chlorphenyl)pyridin;
5-[(trans-5-(4-Butyl-1,3-dioxan-2-yl)äthinyl]-2-(4-chlorphenyl)pyridin;
5-[(trans-5-(4-Pentyl-1,3-dioxan-2-yl)äthinyl]-2-(4-chlorphenyl)pyridin;
5-[(trans-5-(1E-Propenyl)-1,3-dioxan-2-yl)äthinyl]-2-(4-chlorphenyl)pyridin;
5-[(trans-5-(1E-Butenyl)-1,3-dioxan-2-yl)äthinyl]-2-(4-chlorphenyl)pyridin;
5-[(trans-5-(1E-Pentenyl)-1,3-dioxan-2-yl)äthinyl]-2-(4-chlorphenyl)pyridin;
5-[(trans-5-(3-Butenyl)-1,3-dioxan-2-yl)äthinyl]-2-(4-chlorphenyl)pyridin;
5-[(trans-5-(4-Pentenyl)-1,3-dioxan-2-yl)äthinyl]-2-(4-chlorphenyl)pyridin;
5-[(trans-5-Propyl-1,3-dioxan-2-yl)äthinyl]-2-(4-chlor-3-fluorphenyl)pyridin;
5-[(trans-5-Butyl-1,3-dioxan-2-yl)äthinyl]-2-(4-chlor-3-fluorphenyl)pyridin;
5-[(trans-5-Pentyl-1,3-dioxan-2-yl)äthinyl]-2-(4-chlor-3-fluorphenyl)pyridin;
5-[(trans-5-(1E-Propenyl)-1,3-dioxan-2-yl)äthinyl]-2-(4-chlor-3-fluorphenyl)pyridin;
5-[(trans-5-(1E-Butenyl)-1,3-dioxan-2-yl)äthinyl]-2-(4-chlor-3-fluorphenyl)pyridin:
5-[(trans-5-(1E-Pentenyl)-1,3-dioxan-2-yl)äthinyl]-2-(4-chlor-3-fluorphenyl)pyridin;
5-[(trans-5-(3-Butenyl)-1,3-dioxan-2-yl)äthinyl]-2-(4-chlor-3-fluorphenyl)pyridin;
5-[(trans-5-(4-Pentenyl)-1,3-dioxan-2-yl)äthinyl]-2-(4-chlor-3-fluorphenyl)pyridin;
5-[(trans-5-Propyl-1,3-dioxan-2-yl)äthinyl]-2-[4-(trifluormethoxy)phenyl)pyridin;
5-[(trans-5-Butyl-1,3-dioxan-2-yl)äthinyl]-2-[4-(trifluormethoxy)phenyl)pyridin;
5-[(trans-5-Pentyl-1,3-dioxan-2-yl)äthinyl]-2-[4-(trifluormethoxy)phenyl)pyridin;
5-[(trans-5-(1E-Propenyl)-1,3-dioxan-2-yl)äthinyl]-2-[4-(trifluormethoxy)phenyl)pyridin;
5-[(trans-5-Propyl-1,3-dioxan-2-yl)äthinyl]-2-[4-(difluormethoxy)phenyl]pyridin;
5-[(trans-5-Butyl-1,3-dioxan-2-yl)äthinyl]-2-[4-(difluormethoxy)phenyl]pyridin;
5-[(trans-5-(1E-Propenyl)-1,3-dioxan-2-yl)äthinyl]-2-[4-(difluormethoxy)phenyl]pyridin;
5-[(trans-5-(1E-Butenyl-1,3-dioxan-2-yl)äthinyl]-2-[4-(difluormethoxy)phenyl]pyridin;
5-[(trans-5-Butyl-1,3-dioxan-2-yl)äthinyl]-2-[4-(trifluormethyl)phenyl]pyridin;
5-[(trans-5-Propyl-1,3-dioxan-2-yl)äthinyl]-2-(4-fluorphenyl)pyrimidin;
5-[(trans-5-Butyl-1,3-dioxan-2-yl)äthinyl]-2-(4-fluorphenyl)pyrimidin;
5-[(trans-5-Pentyl-1,3-dioxan-2-yl)äthinyl]-2-(4-fluorphenyl)pyrimidin;
5-[(trans-5-(1E-Propenyl)-1,3-dioxan-2-yl)äthinyl]-2-(4-fluorphenyl)pyrimidin;
5-[(trans-5-(1E-Butenyl)-1,3-dioxan-2-yl)äthinyl]-2-(4-fluorphenyl)pyrimidin;
5-[(trans-5-(3-Butenyl)-1,3-dioxan-2-yl)äthinyl]-2-(4-fluorphenyl)pyrimidin;
5-[(trans-5-(4-Pentenyl)-1,3-dioxan-2-yl)äthinyl]-2-(4-fluorphenyl)pyrimidin;
5-[(trans-5-Propyl-1,3-dioxan-2-yl)äthinyl]-2-(3,4-difluorphenyl)pyrimidin, Smp. (C-N) 147,1°C, S_{A}-N 117,0°C, Klp. (N-I) 156°C;
5-[(trans-5-Butyl-1,3-dioxan-2-yl)äthinyl]-2-(3,4-difluorphenyl)pyrimidin;
5-[(trans-5-Pentyl-1,3-dioxan-2-yl)äthinyl]-2-(3,4-difluorphenyl)pyrimidin;
5-[(trans-5-(1E-Propenyl)-1,3-dioxan-2-yl)äthinyl]-2-(3,4-difluorphenyl)pyrimidin, Smp. (C-N) 171,5°C, Klp. (N-I) 201°C;
5-[(trans-5-(1E-Butenyl)-1,3-dioxan-2-yl)äthinyl]-2-(3,4-difluorphenyl)pyrimidin;
5-[(trans-5-(3-Butenyl)-1,3-dioxan-2-yl)äthinyl]-2-(3,4-difluorphenyl)pyrimidin, Smp. (C-N) 136°C, S_{A}-N 124,4°C, Klp. (N-I) 144°C;
5-[(trans-5-(4-Pentenyl)-1,3-dioxan-2-yl)äthinyl]-2-(3,4-difluorphenyl)pyrimidin;
5-[(trans-5-Propyl-1,3-dioxan-2-yl)äthinyl]-2-(4-chlorphenyl)pyrimidin;
5-[(trans-5-Butyl-1,3-dioxan-2-yl)äthinyl]-2-(4-chlorphenyl)pyrimidin;
5-[(trans-5-(1E-Propenyl)-1,3-dioxan-2-yl)äthinyl]-2-(4-chlorphenyl)pyrimidin;
5-[(trans-5-(3-Butenyl)-1,3-dioxan-2-yl)äthinyl]-2-(4-chlorphenyl)pyrimidin;
5-[(trans-5-(4-Pentenyl)-1,3-dioxan-2-yl)äthinyl]-2-(4-chlorphenyl)pyrimidin;
5-[(trans-5-Propyl-1,3-dioxan-2-yl)äthinyl]-2-(4-chlor-3-fluorphenyl)pyrimidin;
5-[(trans-5-Butyl-1,3-dioxan-2-yl)äthinyl]-2-(4-chlor-3-fluorphenyl)pyrimidin;
5-[(trans-5-Pentyl-1,3-dioxan-2-yl)äthinyl]-2-(4-chlor-3-fluorphenyl)pyrimidin;
5-[(trans-5-(1E-Propenyl)-1,3-dioxan-2-yl)äthinyl]-2-(4-chlor-3-fluorphenyl)pyrimidin;
5-[(trans-5-(1E-Butenyl)-1,3-dioxan-2-yl)äthinyl]-2-(4-chlor-3-fluorphenyl)pyrimidin;
5-[(trans-5-(3-Butenyl)-1,3-dioxan-2-yl)äthinyl]-2-(4-chlor-3-fluorphenyl)pyrimidin;
5-[(trans-5-(4-Pentenyl)-1,3-dioxan-2-yl)äthinyl]-2-(4-chlor-3-fluorphenyl)pyrimidin;
5-[(trans-5-Propyl-1,3-dioxan-2-yl)äthinyl]-2-[4-(difluormethoxy)phenyl)pyrimidin;
5-[(trans-5-Butyl-1,3-dioxan-2-yl)äthinyl]-2-[4-(difluormethoxy)phenyl)pyrimidin;
5-[(trans-5-(1E-Propenyl)-1,3-dioxan-2-yl)äthinyl]-2-[4-(difluormethoxy)phenyl)pyrimidin;
4-[(trans-5-Propyl-1,3-dioxan-2-yl)äthinyl]-4'-fluorbiphenyl;
4-[(trans-5-Butyl-1,3-dioxan-2-yl)äthinyl]-4'-fluorbiphenyl;
4-[(trans-5-Pentyl-1,3-dioxan-2-yl)äthinyl]-4'-fluorbiphenyl;
4-[(trans-5-(1E-Propenyl)-1,3-dioxan-2-yl)äthinyl]-4'-fluorbiphenyl;
4-[(trans-5-(1E-Butenyl)-1,3-dioxan-2-yl)äthinyl]-4'-fluorbiphenyl;
4-[(trans-5-(1E-Pentenyl)-1,3-dioxan-2-yl)äthinyl]-4'-fluorbiphenyl;
4-[(trans-5-(3-Butenyl)-1,3-dioxan-2-yl)äthinyl]-4'-fluorbiphenyl;
4-[(trans-5-(4-Pentenyl)-1,3-dioxan-2-yl)äthinyl]-4'-fluorbiphenyl;
4-[(trans-5-Propyl-1,3-dioxan-2-yl)äthinyl]-3',4'-difluorbiphenyl;
4-[(trans-5-Butyl-1,3-dioxan-2-yl)äthinyl]-3',4'-difluorbiphenyl;
4-[(trans-5-Pentyl-1,3-dioxan-2-yl)äthinyl]-3',4'-difluorbiphenyl;
4-[(trans-5-(1E-Propenyl)-1,3-dioxan-2-yl)äthinyl]-3',4'-difluorbiphenyl, Smp. (C-N) 134,4°C, Klp. (N-I) 171°C;
4-[(trans-5-(1E-Butenyl)-1,3-dioxan-2-yl)äthinyl]-3',4'-difluorbiphenyl;
4-[(trans-5-(1E-Pentenyl)-1,3-dioxan-2-yl)äthinyl]-3',4'-difluorbiphenyl;
4-[(trans-5-(3-Butenyl)-1,3-dioxan-2-yl)äthinyl]-3',4'-difluorbiphenyl;
4-[(trans-5-(4-Pentenyl)-1,3-dioxan-2-yl)äthinyl]-3',4'-difluorbiphenyl;
4-[(trans-5-Propyl-1,3-dioxan-2-yl)äthinyl]-4'-chlorbiphenyl;
4-[(trans-5-Butyl-1,3-dioxan-2-yl)äthinyl]-4'-chlorbiphenyl;
4-[(trans-5-Pentyl-1,3-dioxan-2-yl)äthinyl]-4'-chlorbiphenyl;
4-[(trans-5-(1E-Propenyl)-1,3-dioxan-2-yl)äthinyl]-4'-chlorbiphenyl;
4-[(trans-5-(1E-Butenyl)-1,3-dioxan-2-yl)äthinyl]-4'-chlorbiphenyl;
4-[(trans-5-(1E-Pentenyl)-1,3-dioxan-2-yl)äthinyl]-4'-chlorbiphenyl;
4-[(trans-5-(3-Butenyl)-1,3-dioxan-2-yl)äthinyl]-4'-chlorbiphenyl;
4-[(trans-5-(4-Pentenyl)-1,3-dioxan-2-yl)äthinyl]-4'-chlorbiphenyl;
4-[(trans-5-Propyl-1,3-dioxan-2-yl)äthinyl]-4'-chlor-3'-fluorbiphenyl;
4-[(trans-5-Butyl-1,3-dioxan-2-yl)äthinyl]-4'chlor-3'-fluorbiphenyl;
4-[(trans-5-Pentyl-1,3-dioxan-2-yl)äthinyl]-4'-chlor-3'-fluorbiphenyl;
4-[(trans-5-(1E-Propenyl)-1,3-dioxan-2-yl)äthinyl]-4'-chlor-3'-fluorbiphenyl;
4-[(trans-5-(1E-Butenyl)-1,3-dioxan-2-yl)äthinyl]-4'-chlor-3'-fluorbiphenyl;
4-[(trans-5-(1E-Pentenyl)-1,3-dioxan-2-yl)äthinyl]-4'-chlor-3'-fluorbiphenyl;
4-[(trans-5-(3-Butenyl)-1,3-dioxan-2-yl)äthinyl]-4'-chlor-3'-fluorbiphenyl;
4-[(trans-5-(4-Pentenyl)-1,3-dioxan-2-yl)äthinyl]-4'-chlor-3'-fluorbiphenyl;
4-[(trans-5-Propyl-1,3-dioxan-2-yl)äthinyl]-4'-trifluormethoxy)biphenyl;
4-[(trans-5-(1E-Propenyl)-1,3-dioxan-2-yl)äthinyl]-4'-(trifluormethoxy)biphenyl;
4-[(trans-5-(Propyl)-1,3-dioxan-2-yl)äthinyl]-4'-(difluormethoxy)biphenyl;
4-[(trans-5-(Butyl)-1,3-dioxan-2-yl)äthinyl]-4'-(difluormethoxy)biphenyl;
4-[(trans-5-(1E-Propenyl)-1,3-dioxan-2-yl)äthinyl]-4'-(difluormethoxy)biphenyl;
4-[(trans-5-(3-Butenyl)-1,3-dioxan-2-yl)äthinyl]-4'-(difluormethoxy)biphenyl;
4-[(trans-5-(4-Pentenyl)-1,3-dioxan-2-yl)äthinyl]-4'-(difluormethoxy)biphenyl;
4-[(trans-5-Propyl-1,3-dioxan-2-yl)äthinyl]-4'-(trifluormethyl)biphenyl;
4-[(trans-5-(1E-Propenyl)-1,3-dioxan-2-yl)äthinyl]-4'-(trifluormethyl)biphenyl.

### Beispiel 3

In eine Suspension von 2,3 g Methyltriphenylphosphoniumbromid in 25 ml t-Butylmethyläther gibt man in Stickstoffatmosphäre 0,73 g Kalium-t-butylat und rührt 0,5 Stunden bei Raumtemperatur. Dann wird bei 0°C eine Lösung von 0,8 g trans-4-[(4'-Fluor-4-biphenylyl)äthinyl]cyclohexancarboxaldehyd in 25 ml t-Butylmethyläther zugetropft und das Reaktionsgemisch noch 0,5 Stunden bei 0°C und 2,5 Stunden bei Raumtemperatur gerührt, danach mit 50 ml gesättigter Natriumhydrogencarbonat-Lösung und mit 50 ml gesättigter Kochsalzlösung gewaschen, über Natriumsulfat getrocknet, filtriert und eingeengt. Der Rückstand wird an Kieselgel mit Hexan chromatographiert. Das erhaltene 4-[(trans-4-Vinylcyclohexyl)äthinyl]-4'-fluorbiphenyl wird zur Reinigung aus Hexan umkristallisiert; Smp. (C-N) 133,9°C, Klp. (N-I) 170°C.

Der als Ausgangsmaterial verwendete trans-4-[(4'-Fluor-4-biphenylyl)äthinyl]cyclohexancarboxaldehyd wurde folgendermassen hergestellt:
a) Ein Gemisch aus 1,03 g 8-Aethinyl-1,4-dioxaspiro[4.5]decan, 1,197 g 4-Brom-4'-fluorbiphenyl, 0,11 g Tetrakis(triphenylphosphin)palladium, 0,041 g Kupfer(I)bromid und 45 ml Triäthylamin wurde 4,5 Stunden zum Sieden erhitzt. Nach dem Erkalten wurde mit Diäthyläther verdünnt, zweimal mit Wasser gewaschen, über Natriumsulfat getrocknet, filtriert und eingeengt. Das erhaltene rohe 8-[(4'-Fluor-4-biphenylyl)äthinyl]-1,4-dioxaspiro[4.5]decan (1,82 g) wurde durch Chromatographie an 35 g Kieselgel mit Hexan/Aethylacetat (Vol. 9:1) gereinigt. Ausbeute 1,30 g.
b) Eine Lösung von 1,30 g 8-[(4'-Fluor-4-biphenylyl)äthinyl]-1,4-dioxaspiro[4.5]decan in 30 ml Tetrahydrofuran wurde mit 3,5 ml 3N Salzsäure 24 Stunden bei 55°C gerührt. Das Reaktionsgemisch wurde mit Diäthyläther verdünnt, mit gesättigter Natriumhydrogencarbonat-Lösung und mit gesättigter Kochsalzlösung gewaschen, über Natriumsulfat getrocknet, filtriert und eingeengt. Es resultierten 1,11 g 4-[(4'-Fluor-4-biphenylyl)äthinyl]cyclohexanon.
c) Zu einer Suspension von 1,60 g (Methoxymethyl)triphenylphosphoniumchlorid in 40 ml t-Butylmethyläther gab man in Stickstoffatmosphäre 0,52 g Kalium-t-butylat und rührte die orangerote Suspension 0,5 Stunden bei Raumtemperatur. Bei 5°C wurde dann eine Lösung von 1,11 g 4-[(4'-Fluor-4-biphenylyl)äthinyl]cyclohexanon in 30 ml Tetrahydrofuran zugetropft. Das Reaktionsgemisch wurde noch 0,5 Stunden bei 5°C und 2 Stunden bei Raumtemperatur gerührt, mit Diäthyläther verdünnt, mit 50 ml gesättigter Natriumhydrogencarbonat-Lösung und mit 50 ml gesättigter Kochsalzlösung gewaschen, über Natriumsulfat getrocknet, filtriert und eingeengt. Chromatographie des Rückstands an Kieselgel mit Hexan/Aethylacetat (Vol. 19:1) lieferte 0,95 g 4'-Fluor-4-[[4-(methoxymethyliden)cyclohexyl]äthinyl]biphenyl.
d) Eine Lösung von 0,95 g 4'-Fluor-4-[[4-methoxymethylidencyclohexyl]äthinyl]biphenyl in 40 ml Toluol wurde mit 4 ml Ameisensäure versetzt und über Nacht bei Raumtemperatur gerührt. Die Ameisensäure-Phase wurde abgetrennt und die Toluol-Phase mit Wasser neutral gewaschen, über Natriumsulfat getrocknet, filtriert und eingeengt. Der erhaltene rohe 4-[(4'-Fluor-4-biphenylyl)äthinyl]cyclohexancarboxaldehyd wurde zur Ringisomerisierung in 35 ml Aethanol gelöst, mit 8,2 ml 0,1N alkoholischer Kaliumhydroxid-Lösung versetzt und 2 Stunden bei Raumtemperatur gerührt. Das Reaktionsgemisch wurde mit Diäthyläther verdünnt, mit gesättigter Natriumhydrogencarbonat-Lösung und mit Wasser gewaschen, über Natriumsulfat getrocknet, filtriert und eingeengt. Es resultierten 0,80 g trans-4-[(4'-Fluor-4-biphenylyl)äthinyl]cyclohexancarboxaldehyd.

In analoger Weise können hergestellt werden:
4-[(trans-4-Vinylcyclohexyl)äthinyl]-3',4'-difluorbiphenyl;
4-[(trans-4-Vinylcyclohexyl)äthinyl]-4'-chlorbiphenyl;
4-[(trans-4-Vinylcyclohexyl)äthinyl]-4'-chlor-3'-fluorbiphenyl;
4-[(trans-4-Vinylcyclohexyl)äthinyl]-4'-brombiphenyl;
4-[(trans-4-Vinylcyclohexyl)äthinyl]-4'-(trifluormethoxy)biphenyl;
4-[(trans-4-Vinylcyclohexyl)äthinyl]-4'-(difluormethoxy)-3'-fluorbiphenyl;
4-[(trans-4-Vinylcyclohexyl)äthinyl]-4'-(trifluormethyl)biphenyl;
5-[(trans-4-Vinylcyclohexyl)äthinyl]-2-(4-fluorphenyl)pyridin;
5-[(trans-4-Vinylcyclohexyl)äthinyl]-2-(3,4-difluorphenyl)pyridin;
5-[(trans-4-Vinylcyclohexyl)äthinyl]-2-(4-chlorphenyl)pyridin;
5-[(trans-4-Vinylcyclohexyl)äthinyl]-2-(4-chlor-3-fluorphenyl)pyridin;
5-[(trans-4-Vinylcyclohexyl)äthinyl]-2-(4-fluorphenyl)pyrimidin;
5-[(trans-4-Vinylcyclohexyl)äthinyl]-2-(3,4-difluorphenyl)pyrimidin;
5-[(trans-4-Vinylcyclohexyl)äthinyl]-2-(4-chlorphenyl)pyrimidin;
5-[(trans-4-Vinylcyclohexyl)äthinyl]-2-(4-chlor-3-fluorphenyl)pyrimidin.

### Beispiel 4

In analoger Weise zu Beispiel 3 werden aus 2,4 g Aethyltriphenylphosphoniumbromid, 0,73 g Kalium-t-butylat und 0,8 g trans-4-[(4'-Fluor-4-biphenylyl)äthinyl]cyclohexancarboxaldehyd in t-Butylmethyläther nach Aufarbeitung und Chromatographie 0,68 g rohes 4-[trans-4-(1Z-Propenylcyclohexyl)äthinyl]-4'-fluorbiphenyl erhalten. Dieses wird zur Isomerisierung in 30 ml Aethanol gelöst und mit 560 mg Benzolsulfinsäure versetzt. Das Gemisch wird über Nacht auf 60°C erwärmt und dann eingeengt. Der Rückstand wird in Diäthyläther und Wasser aufgenommen, die Wasserphase abgetrennt und die organische Phase mit Wasser neutral gewaschen, über Natriumsulfat getrocknet, filtriert und eingeengt. Mehrfache Umkristallisation des Rückstands aus Hexan liefert reines 4-[trans-4-(1E-Propenylcyclohexyl)äthinyl]-4'-fluorbiphenyl.

In analoger Weise können hergestellt werden:
4-[[trans-4-(1E-Butenyl)cyclohexyl]äthinyl]-4'-fluorbiphenyl;
4-[[trans-4-(1E-Pentenyl)cyclohexyl]äthinyl]-4'-fluorbiphenyl;
4-[[trans-4-(1E-Propenyl)cyclohexyl]äthinyl]-3',4'-difluorbiphenyl;
4-[[trans-4-(1E-Butenyl)cyclohexyl]äthinyl]-3',4'-difluorbiphenyl;
4-[[trans-4-(1E-Pentenyl)cyclohexyl]äthinyl]-3',4'-difluorbiphenyl;
4-[[trans-4-(1E-Propenyl)cyclohexyl]äthinyl]-4'-chlorbiphenyl;
4-[[trans-4-(1E-Butenyl)cyclohexyl]äthinyl]-4'-chlorbiphenyl;
4-[[trans-4-(1E-Propenyl)cyclohexyl]äthinyl]-4'-chlor-3'-fluorbiphenyl;
4-[[trans-4-(1E-Butenyl)cyclohexyl]äthinyl]-4'-chlor-3'-fluorbiphenyl;
4-[[trans-4-(1E-Pentenyl)cyclohexyl]äthinyl]-4'-chlor-3'-fluorbiphenyl;
5-[[trans-4-(1E-Propenyl)cyclohexyl]äthinyl]-2-(4-fluorphenyl)pyridin;
5-[[trans-4-(1E-Butenyl)cyclohexyl]äthinyl]-2-(4-fluorphenyl)pyridin;
5-[[trans-4-(1E-Pentenyl)cyclohexyl]äthinyl]-2-(4-fluorphenyl)pyridin;
5-[[trans-4-(1E-Propenyl)cyclohexyl]äthinyl]-2-(3,4-difluorphenyl)pyridin;
5-[[trans-4-(1E-Butenyl)cyclohexyl]äthinyl]-2-(3,4-difluorphenyl)pyridin;
5-[[trans-4-(1E-Pentenyl)cyclohexyl]äthinyl]-2-(3,4-difluorphenyl)pyridin;
5-[[trans-4-(1E-Propenyl)cyclohexyl]äthinyl]-2-(4-chlorphenyl)pyridin;
5-[[trans-4-(1E-Butenyl)cyclohexyl]äthinyl]-2-(4-chlorphenyl)pyridin;
5-[[trans-4-(1E-Propenyl)cyclohexyl]äthinyl]-2-(4-chlor-3-fluorphenyl)pyridin;
5-[[trans-4-(1E-Butenyl)cyclohexyl]äthinyl]-2-(4-chlor-3-fluorphenyl)pyridin;
5-[[trans-4-(1E-Pentenyl)cyclohexyl]äthinyl]-2-(4-chlor-3-fluorphenyl)pyridin;
5-[[trans-4-(1E-Propenyl)cyclohexyl]äthinyl]-2-(4-fluorphenyl)pyrimidin;
5-[[trans-4-(1E-Butenyl)cyclohexyl]äthinyl]-2-(4-fluorphenyl)pyrimidin;
5-[[trans-4-(1E-Propenyl)cyclohexyl]äthinyl]-2-(3,4-difluorphenyl)pyrimidin;
5-[[trans-4-(1E-Butenyl)cyclohexyl]äthinyl]-2-(3,4-difluorphenyl)pyrimidin;
5-[[trans-4-(1E-Propenyl)cyclohexyl]äthinyl]-2-(4-chlorphenyl)pyrimidin;
5-[[trans-4-(1E-Butenyl)cyclohexyl]äthinyl]-2-(4-chlorphenyl)pyrimidin;
5-[[trans-4-(1E-Propenyl)cyclohexyl]äthinyl]-2-(4-chlor-3-fluorphenyl)pyrimidin;
5-[[trans-4-(1E-Butenyl)cyclohexyl]äthinyl]-2-(4-chlor-3-fluorphenyl)pyrimidin.

### Beispiel 5

Aus 1,15 g Methyltriphenylphosphoniumbromid 0,37 g Kalium-t-butylat und 0,44 g 3-[trans-4-[(4'-Fluor-4-biphenylyl)äthinyl]cyclohexyl]propanal wird analog wie in Beispiel 3 beschrieben 4-[[trans-4-(3-Butenyl)cyclohexyl]äthinyl]-4'-fluorbiphenyl erhalten.

Das als Ausgangsmaterial verwendete 3-[trans-4-[(4'-Fluor-4-biphenylyl)äthinyl]cyclohexyl]propanal kann ausgehend von trans-4-[(4'-Fluor-4-biphenylyl)äthinyl]cyclohexancarboxaldehyd durch zweimalige Durchführung der Reaktionssequenz Wittig-Reaktion mit (Methoxymethyl)triphenylphosphoniumchlorid/Kalium-t-butylat (siehe Beispiel 2c) und Hydrolyse der erhaltenen Methoxyvinyl-Verbindung mit Ameisensäure zum homologen Aldehyd (siehe Beispiel 2d).

In analoger Weise können hergestellt werden:
4-[[trans-4-(3-Butenyl)cyclohexyl]äthinyl]-3',4'-difluorbiphenyl;
4-[[trans-4-(3-Butenyl)cyclohexyl]äthinyl]-4'-chlorbiphenyl;
4-[[trans-4-(3-Butenyl)cyclohexyl]äthinyl]-4'-chlor-3'-fluorbiphenyl;
4-[[trans-4-(3-Butenyl)cyclohexyl]äthinyl]-4'-(trifluormethoxy)biphenyl;
4-[[trans-4-(3-Butenyl)cyclohexyl]äthinyl]-4'-(difluormethoxy)biphenyl;
5-[[trans-4-(3-Butenyl)cyclohexyl]äthinyl]-2-(4-fluorphenyl)pyridin;
5-[[trans-4-(3-Butenyl)cyclohexyl]äthinyl]-2-(3,4-difluorphenyl)pyridin;
5-[[trans-4-(3-Butenyl)cyclohexyl]äthinyl]-2-(4-chlorphenyl)pyridin;
5-[[trans-4-(3-Butenyl)cyclohexyl]äthinyl]-2-(4-chlor-3-fluorphenyl)pyridin;
5-[[trans-4-(3-Butenyl)cyclohexyl]äthinyl]-2-(4-fluorphenyl)pyrimidin;
5-[[trans-4-(3-Butenyl)cyclohexyl]äthinyl]-2-(3,4-difluorphenyl)pyrimidin;
5-[[trans-4-(3-Butenyl)cyclohexyl]äthinyl]-2-(4-chlorphenyl)pyrimidin;
5-[[trans-4-(3-Butenyl)cyclohexyl]äthinyl]-2-(4-chlor-3-fluorphenyl)pyrimidin.

Wird 3-[trans-4-[(4'-Fluor-4-biphenylyl)äthinyl]cyclohexyl]propanal einer zusätzlichen Homologisierungssequenz Wittig-Reaktion mit (Methoxymethyl)triphenylphosphoniumchlorid/Kalium-t-butylat und Hydrolyse mit Ameisensäure vor der abschliessenden Wittigreaktion mit Methyltriphenylphosphoniumbromid/Kalium-t-butylat unterzogen, so wird 4-[[trans-4-(4-Pentenyl)cyclohexyl]äthinyl]-4'-fluorbiphenyl erhalten.

In analoger Weise können hergestellt werden:
4-[[trans-4-(4-Pentenyl)cyclohexyl]äthinyl]-3',4'-difluorbiphenyl;
4-[[trans-4-(4-Pentenyl)cyclohexyl]äthinyl]-4'-chlorbiphenyl;
4-[[trans-4-(4-Pentenyl)cyclohexyl]äthinyl]-4'-chlor-3'-fluorbiphenyl;
5-[[trans-4-(4-Pentenyl)cyclohexyl]äthinyl]-2-(4-fluorphenyl)pyridin;
5-[[trans-4-(4-Pentenyl)cyclohexyl]äthinyl]-2-(3,4-difluorphenyl)pyridin;
5-[[trans-4-(4-Pentenyl)cyclohexyl]äthinyl]-2-(4-chlorphenyl)pyridin;
5-[[trans-4-(4-Pentenyl)cyclohexyl]äthinyl]-2-(4-chlor-3-fluorphenyl)pyridin;
5-[[trans-4-(4-Pentenyl)cyclohexyl]äthinyl]-2-(4-fluorphenyl)pyrimidin;
5-[[trans-4-(4-Pentenyl)cyclohexyl]äthinyl]-2-(3,4-difluorphenyl)pyrimidin;
5-[[trans-4-(4-Pentenyl)cyclohexyl]äthinyl]-2-(4-chlorphenyl)pyrimidin;
5-[[trans-4-(4-Pentenyl)cyclohexyl]äthinyl]-2-(4-chlor-3-fluorphenyl)pyrimidin.

### Beispiel 6

0,15 g Natriumhydrid-Suspension in Oel (∼55%) wird mit Pentan gewaschen und mit 5 ml Tetrahydrofuran versetzt. Eine Lösung von 0,36 g 3-[trans-4-[(4'-Fluor-4-biphenylyl)äthinyl]cyclohexyl]propanol in 5 ml Tetrahydrofuran wird zugetropft und das Reaktionsgemisch 0,5 Stunden gerührt, bevor 0,2 ml Methyljodid zugegeben werden. Die Suspension wird 1 Stunde bei 62°C gerührt. Nach dem Erkalten wird mit 40 ml Diäthyläther verdünnt und mit Wasser neutral gewaschen, über Natriumsulfat getrocknet und eingeengt. Chromatographie des Rückstands an Hexan/Aethylacetat (Vol. 99:1) und Umkristallisation aus Hexan liefert reines 4'-[[trans-4-(3-Methoxypropyl)cyclohexyl]äthinyl]-4'-fluorbiphenyl.

Das als Ausgangsmaterial verwendete 3-[trans-4-[(4'-Fluor-4-biphenylyl)äthinyl]cyclohexyl]propanol kann wie folgt hergestellt werden:

Eine Lösung von 0,44 g 3-[trans-4-[(4'-Fluor-4-biphenylyl)äthinyl]cyclohexyl]propanal in 20 ml Methanol/Wasser (Vol. 4:1) und 15 ml Dioxan wird portionenweise mit 0,10 g Natriumborhydrid versetzt. Das Reaktionsgemisch wird 0,75 Stunden bei Raumtemperatur gerührt und eingeengt. Der Rückstand wird mit Wasser versetzt und mit Diäthyläther extrahiert. Die Aetherphase wird mit Wasser gewaschen, über Natriumsulfat getrocknet, filtriert und eingedampft. Es werden 0,36 g 3-[trans-4-[(4'-Fluor-4-biphenylyl)äthinyl]cyclohexyl]propanol erhalten.

### Beispiel 7

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung (V₁₀) und die Ansprechzeiten (tₒₙ und t_{off}) wurden in einer TN-Zelle (low bias tilt) mit 8 µm Plattenabstand bei 22°C gemessen; als Betriebsspannung wurde der 2,5-fache Wert der Schwellenspannung (V₁₀) gewählt. Die entsprechenden Daten für 4-(trans-4-Pentylcyclohexyl)benzonitril betragen: Klp. (N-I) 54,6°C, V₁₀ = 1,62V, tₒₙ = 22 ms, t_{off} = 42ms, Δn = 0,120.

### BM-1

- 90 Gew.%: 4-(trans-4-Pentylcyclohexyl)benzonitril
- 10 Gew.%: 4-[(trans-4-Propylcyclohexyl)äthinyl]-4'-fluorbiphenyl
Klp. (N-I) 61,4°C, V₁₀ = 1,58V, tₒₙ = 28 ms, t_{off} = 42 ms, Δn = 0,132

### BM-2

- 80 Gew.%: 4-(trans-4-Pentylcyclohexyl)benzonitril
- 20 Gew.%: 4-[(trans-4-Propylcyclohexyl)äthinyl]-4'-fluorbiphenyl
Klp. (N-I) 69,9°C, V₁₀ = 1,63V, tₒₙ = 30 ms, t_{off} = 45 ms, Δn = 0,144

### BM-3

- 90 Gew.%: 4-(trans-4-Pentylcyclohexyl)benzonitril
- 10 Gew.%: 4-[(trans-4-Butylcyclohexyl)äthinyl]-4'-fluorbiphenyl
Klp. (N-I) 61,0°C, V₁₀ = 1,60V, tₒₙ = 28 ms, t_{off} = 45 ms, Δn = 0,130

### BM-4

- 90 Gew.%: 4-(trans-4-Pentylcyclohexyl)benzonitril
- 10 Gew.%: 4-[(trans-4-Propylcyclohexyl)äthinyl]-3',4'-difluorbiphenyl
Klp. (N-I) 58,2°C, V₁₀ = 1,43V, tₒₙ = 32 ms, t_{off} = 45 ms, Δn = 0,130

### BM-5

- 80 Gew.%: 4-(trans-4-Pentylcyclohexyl)benzonitril
- 20 Gew.%: 4-[(trans-4-Propylcyclohexyl)äthinyl]-3',4'-difluorbiphenyl
Klp. (N-I) 61,4°C, V₁₀ = 1,42V, tₒₙ = 38 ms, t_{off} = 52 ms, Δn = 0,135

### BM-6

- 90 Gew.%: 4-(trans-4-Pentylcyclohexyl)benzonitril
- 10 Gew.%: 4-[(trans-4-Propylcyclohexyl)äthinyl]-2-(3,4-difluorphenyl)pyridin
Klp. (N-I) 57,5°C, V₁₀ = 1,43V, tₒₙ = 31 ms, t_{off} = 49 ms, Δn = 0,130

### BM-7

- 90 Gew.%: 4-(trans-4-Pentylcyclohexyl)benzonitril
- 10 Gew.%: 4-[(trans-4-Butylcyclohexyl)äthinyl]-4'-chlorbiphenyl
Klp. (N-I) 63.8°C, V₁₀ = 1.66V, tₒₙ = 26 ms, t_{off} = 46 ms, Δn = 0.134

### BM-8

- 80 Gew.%: 4-(trans-4-Pentylcyclohexyl)benzonitril
- 20 Gew.%: 4-[(trans-4-Butylcyclohexyl)äthinyl]-4'-chlorbiphenyl
Klp. (N-I) 73.3°C, V₁₀ = 1.72V, tₒₙ = 29 ms, t_{off} = 49 ms, Δn = 0.142

### BM-9

- 90 Gew.%: 4-(trans-4-Pentylcyclohexyl)benzonitril
- 10 Gew.%: 4-[(trans-4-Propylcyclohexyl)äthinyl]-4'-chlor-3'-fluorbiphenyl
Klp. (N-I) 60.2°C, V₁₀ = 1.56V, tₒₙ = 30 ms, t_{off} = 49 ms, Δn = 0.132

### BM-10

- 80 Gew.%: 4-(trans-4-Pentylcyclohexyl)benzonitril
- 20 Gew.%: 4-[(trans-4-Propylcyclohexyl)äthinyl]-4'-chlor-3'-fluorbiphenyl
Klp. (N-I) 65.8°C, V₁₀ = 1.60V, tₒₙ = 33 ms, t_{off} = 55 ms, Δn = 0.138

### BM-11

- 90 Gew.%: 4-(trans-4-Pentylcyclohexyl)benzonitril
- 10 Gew.%: 4-[(trans-4-Butylcyclohexyl)äthinyl]-4'-chlor-3'-fluorbiphenyl
Klp. (N-I) 59.6°C, V₁₀ = 1.55V, tₒₙ = 29 ms, t_{off} = 50 ms, Δn = 0.130

### BM-12

- 80 Gew.%: 4-(trans-4-Pentylcyclohexyl)benzonitril
- 20 Gew.%: 4-[(trans-4-Butylcyclohexyl)äthinyl]-4'-chlor-3'-fluorbiphenyl
Klp. (N-I) 63.8°C, V₁₀ = 1.53V, tₒₙ = 39 ms, t_{off} = 66 ms, Δn = 0.137

### BM-13

- 80 Gew.%: 4-(trans-4-Pentylcyclohexyl)benzonitril
- 20 Gew.%: 4-[(trans-4-Propylcyclohexyl)äthinyl]-2-(3,4-difluorphenyl)pyridin
Klp. (N-I) 60,5°C, V₁₀ = 1,34V, tₒₙ = 40 ms, t_{off} = 58 ms, Δn = 0,137

### BM-14

- 90 Gew.%: 4-(trans-4-Pentylcyclohexyl)benzonitril
- 10 Gew.%: 5-[(trans-4-Propylcyclohexyl)äthinyl]-2-(4-chlor-3-fluorphenyl)pyridin
Klp. (N-I) 60,1°C, V₁₀ = 1,49V, tₒₙ = 27 ms, t_{off} = 45 ms, Δn = 0,134

### BM-15

- 80 Gew.%: 4-(trans-4-Pentylcyclohexyl)benzonitril
- 20 Gew.%: 5-[(trans-4-Propylcyclohexyl)äthinyl]-2-(4-chlor-3-fluorphenyl)pyridin
Klp. (N-I) 65,6°C, V₁₀ = 1,47V, tₒₙ = 37 ms, t_{off} = 58 ms, Δn = 0,146

### BM-16

- 90 Gew.%: 4-(trans-4-Pentylcyclohexyl)benzonitril
- 10 Gew.%: 5-[(trans-4-Propylcyclohexyl)äthinyl]-2-(4-fluorphenyl)pyrimidin
Klp. (N-I) 62.0 °C, V₁₀ = 1.56 V, tₒₙ = 29 ms, t_{off} = 48 ms, Δn = 0.133

### BM-17

- 80 Gew.%: 4-(trans-4-Pentylcyclohexyl)benzonitril
- 20 Gew.%: 5-[(trans-4-Propylcyclohexyl)äthinyl]-2-(4-fluorphenyl)pyrimidin
Klp. (N-I) 69.7 °C, V₁₀ = 1.56 V, tₒₙ = 30 ms, t_{off} = 49 ms, Δn = 0.144

### BM-18

- 90 Gew.%: 4-(trans-4-Pentylcyclohexyl)benzonitril
- 10 Gew.%: 5-[(trans-4-Butylcyclohexyl)äthinyl]-2-(4-fluorphenyl)pyrimidin
Klp. (N-I) 61.7 °C, V₁₀ = 1.54 V, tₒₙ = 27 ms, t_{off} = 47 ms, Δn = 0.131

### BM-19

- 80 Gew.%: 4-(trans-4-Pentylcyclohexyl)benzonitril
- 20 Gew.%: 5-[(trans-4-Butylcyclohexyl)äthinyl]-2-(4-fluorphenyl)pyrimidin
Klp. (N-I) 69.3 °C, V₁₀ = 1.51 V, tₒₙ = 32 ms, t_{off} = 52 ms, Δn = 0.141

### BM-20

- 90 Gew.%: 4-(trans-4-Pentylcyclohexyl)benzonitril
- 10 Gew.%: 5-[(trans-4-Propylcyclohexyl)äthinyl]-2-(3,4-difluorphenyl)pyrimidin
Klp. (N-I) 59.1 °C, V₁₀ = 1.48 V, tₒₙ = 31 ms, t_{off} = 47 ms, Δn = 0.130

### BM-21

- 80 Gew.%: 4-(trans-4-Pentylcyclohexyl)benzonitril
- 20 Gew.%: 5-[(trans-4-Propylcyclohexyl)äthinyl]-2-(3,4-difluorphenyl)pyrimidin
Klp. (N-I) 63.9 °C, V₁₀ = 1.45 V, tₒₙ = 32 ms, t_{off} = 52 ms

### BM-22

- 90 Gew.%: 4-(trans-4-Pentylcyclohexyl)benzonitril
- 10 Gew.%: 5-[(trans-4-Butylcyclohexyl)äthinyl]-2-(3,4-difluorphenyl)pyrimidin
Klp. (N-I) 58.9 °C, V₁₀ = 1.44 V, tₒₙ = 31 ms, t_{off} = 49 ms, Δn = 0.129

### BM-23

- 80 Gew.%: 4-(trans-4-Pentylcyclohexyl)benzonitril
- 20 Gew.%: 5-[(trans-4-Butylcyclohexyl)äthinyl]-2-(3,4-difluorphenyl)pyrimidin
Klp. (N-I) 63.8 °C, V₁₀ = 1.41 V, tₒₙ = 34 ms, t_{off} = 56 ms, Δn = 0.135

### BM-24

- 90 Gew.%: 4-(trans-4-Pentylcyclohexyl)benzonitril
- 10 Gew.%: 5-[(trans-4-Propylcyclohexyl)äthinyl]-2-(4-chlor-3-fluorphenyl)pyrimidin
Klp. (N-I) 61.3 °C, V₁₀ = 1.44 V, tₒₙ = 31 ms, t_{off} = 48 ms, Δn = 0.134

### BM-25

- 80 Gew.%: 4-(trans-4-Pentylcyclohexyl)benzonitril
- 20 Gew.%: 5-[(trans-4-Propylcyclohexyl)äthinyl]-2-(4-chlor-3-fluorphenyl)pyrimidin
Klp. (N-I) 67.0 °C, V₁₀ = 1.47 V, tₒₙ = 35 ms, t_{off} = 56 ms, Δn = 0.147

### BM-26

- 90 Gew.%: 4-(trans-4-Pentylcyclohexyl)benzonitril
- 10 Gew.%: 5-[(trans-4-Butylcyclohexyl)äthinyl]-2-(4-chlor-3-fluorphenyl)pyrimidin
Klp. (N-I) 61.3 °C, V₁₀ = 1.49 V, tₒₙ = 29 ms, t_{off} = 49 ms, Δn = 0.131

### BM-27

- 80 Gew.%: 4-(trans-4-Pentylcyclohexyl)benzonitril
- 20 Gew.%: 5-[(trans-4-Butylcyclohexyl)äthinyl]-2-(4-chlor-3-fluorphenyl)pyrimidin
Klp. (N-I) 69.3 °C, V₁₀ = 1.47 V, tₒₙ = 33 ms, t_{off} = 57 ms

### BM-28

- 90 Gew.%: 4-(trans-4-Pentylcyclohexyl)benzonitril
- 10 Gew.%: 4-[(trans-5-(1E-Propenyl)-1,3-dioxan-2-yl)äthinyl]-3',4'-difluorbiphenyl
Klp. (N-I) 57.7 °C, V₁₀ = 1.47 V, tₒₙ = 31 ms, t_{off} = 45 ms, Δn = 0.124

### BM-29

- 90 Gew.%: 4-(trans-4-Pentylcyclohexyl)benzonitril
- 10 Gew.%: 5-[(trans-5-Propyl-1,3-dioxan-2-yl)äthinyl]-2-(3,4-difluorphenyl)pyridin
Klp. (N-I) 55.5 °C, V₁₀ = 1.42 V, tₒₙ = 36 ms, t_{off} = 56 ms, Δn = 0.128

### BM-30

- 90 Gew.%: 4-(trans-4-Pentylcyclohexyl)benzonitril
- 10 Gew.%: 4-(1-Hexinyl)-4'-fluorbiphenyl
Klp. (N-I) 44.0 °C, V₁₀ = 1.30 V, tₒₙ = 31 ms, t_{off} = 50 ms, Δn = 0.121

### BM-31

- 80 Gew.%: 4-(trans-4-Pentylcyclohexyl)benzonitril
- 20 Gew.%: 4-(1-Hexinyl)-4'-fluorbiphenyl
Klp. (N-I) 34.5 °C, tₒₙ = 30 ms, t_{off} = 61 ms, Δn = 0.111

### BM-32

- 90 Gew.%: 4-(trans-4-Pentylcyclohexyl)benzonitril
- 10 Gew.%: 5-(1-Hexinyl)-2-(3,4-difluorphenyl)pyridin
Klp. (N-I) 42.0 °C, V₁₀ = 1.28 V, tₒₙ = 30 ms, t_{off} = 50 ms, Δn = 0.116

### BM-33

- 80 Gew.%: 4-(trans-4-Pentylcyclohexyl)benzonitril
- 20 Gew.%: 5-(1-Hexinyl)-2-(3,4-difluorphenyl)pyridin
Klp. (N-I) 30.3 °C, V₁₀ = 1.01 V, tₒₙ = 38 ms, t_{off} = 66 ms, Δn = 0.099

### BM-34

- 90 Gew.%: 4-(trans-4-Pentylcyclohexyl)benzonitril
- 10 Gew.%: 5-(1-Pentinyl)-2-(4-chlor-3-fluorphenyl)pyridin
Klp. (N-I) 47.0 °C, V₁₀ = 1.35 V, tₒₙ = 29 ms, t_{off} = 47 ms, Δn = 0.126

### BM-35

- 80 Gew.%: 4-(trans-4-Pentylcyclohexyl)benzonitril
- 20 Gew.%: 5-(1-Pentinyl)-2-(4-chlor-3-fluorphenyl)pyridin
Klp. (N-I) 39.5 °C, V₁₀ = 1.16 V, tₒₙ = 34 ms, t_{off} = 56 ms, Δn = 0.128

### BM-36

- 90 Gew.%: 4-(trans-4-Pentylcyclohexyl)benzonitril
- 10 Gew.%: 5-(1-Pentinyl)-2-(3,4-difluorphenyl)pyrimidin
Klp. (N-I) 46.2 °C, V₁₀ = 1.31 V, tₒₙ = 28 ms, t_{off} = 45 ms, Δn = 0.124

### BM-37

- 80 Gew.%: 4-(trans-4-Pentylcyclohexyl)benzonitril
- 20 Gew.%: 5-(1-Pentinyl)-2-(3,4-difluorphenyl)pyrimidin
Klp. (N-I) 38.9 °C, V₁₀ = 1.11 V, tₒₙ = 31 ms, t_{off} = 49 ms, Δn = 0.123

### BM-38

- 90 Gew.%: 4-(trans-4-Pentylcyclohexyl)benzonitril
- 10 Gew.%: 5-(1-Hexinyl)-2-(3,4-difluorphenyl)pyrimidin
Klp. (N-I) 44.4 °C, V₁₀ = 1.26 V, tₒₙ = 31 ms, t_{off} = 49 ms, Δn = 0.120

### BM-39

- 80 Gew.%: 4-(trans-4-Pentylcyclohexyl)benzonitril
- 20 Gew.%: 5-(1-Hexinyl)-2-(3,4-difluorphenyl)pyrimidin
Klp. (N-I) 35.4 °C, V₁₀ = 1.15 V, tₒₙ = 28 ms, t_{off} = 55 ms, Δn = 0.115

### BM-40

- 90 Gew.%: 4-(trans-4-Pentylcyclohexyl)benzonitril
- 10 Gew.%: 5-(1-Hexinyl)-2-(4-fluorphenyl)pyrimidin
Klp. (N-I) 47.0 °C, V₁₀ = 1.35 V, tₒₙ = 27 ms, t_{off} = 43 ms, Δn = 0.124

### BM-41

- 80 Gew.%: 4-(trans-4-Pentylcyclohexyl)benzonitril
- 20 Gew.%: 5-(1-Hexinyl)-2-(4-fluorphenyl)pyrimidin
Klp. (N-I) 40.2 °C, V₁₀ = 1.20 V, tₒₙ = 29 ms, t_{off} = 48 ms, Δn = 0.125

### BM-42

- 90 Gew.%: 4-(trans-4-Pentylcyclohexyl)benzonitril
- 10 Gew.%: 5-(1-Pentinyl)-2-(4-chlor-3-fluorphenyl)pyrimidin
Klp. (N-I) 48.1 °C, V₁₀ = 1.36 V, tₒₙ = 27 ms, t_{off} = 44 ms, Δn = 0.125

### BM-43

- 80 Gew.%: 4-(trans-4-Pentylcyclohexyl)benzonitril
- 20 Gew.%: 5-(1-Pentinyl)-2-(4-chlor-3-fluorphenyl)pyrimidin
Klp. (N-I) 44.1 °C, V₁₀ = 1.20 V, tₒₙ = 30 ms, t_{off} = 50 ms, Δn = 0.127

### BM-44

- 90 Gew.%: 4-(trans-4-Pentylcyclohexyl)benzonitril
- 10 Gew.%: 4-[(trans-4-Propylcyclohexyl)äthinyl]-4'-difluormethoxy)-3',5'-difluorbiphenyl
Klp. (N-I) 57,6°C, V₁₀ = 1,58 V, tₒₙ = 29 ms, t_{off} = 50 ms, Δn = 0,128.

### BM-45

- 80 Gew.%: 4-(trans-4-Pentylcyclohexyl)benzonitril
- 20 Gew.%: 4-[(trans-4-Propylcyclohexyl)äthinyl]-4'-difluormethoxy)-3',5'-difluorbiphenyl
Klp. (N-I) 60,9°C, V₁₀ = 1,51 V, tₒₙ = 35 ms, t_{off} = 59 ms, Δn = 0,134.

## Patentansprüche

1. Verbindungen der allgemeinen Formel 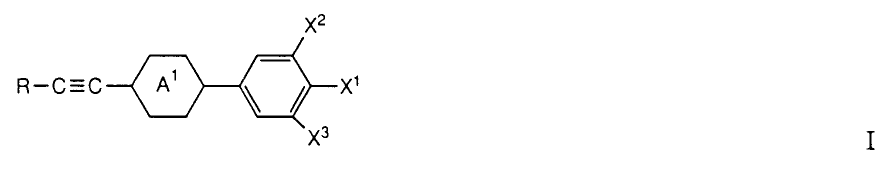 worin
R eine Alkylgruppe mit 1 bis 12 Kohlenstoffatomen oder eine Gruppe der Formel 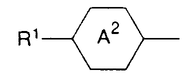 bedeutet;
Ring A¹ unsubstituiertes oder mit Fluor substituiertes 1,4-Phenylen, Pyridin-2,5-diyl oder Pyrimidin-2,5-diyl darstellt;
Ring A² trans-1,4-Cyclohexylen oder trans-1,3-Dioxan-2,5-diyl bedeutet;
R¹ eine Alkyl-, Alkenyl-, oder Alkoxyalkylgruppe mit 1 bis 12, bzw. 2 bis 12 Kohlenstoffatomen darstellt;
X¹ Fluor, Chlor, Brom, Trifluormethyl, Trifluormethoxy oder Difluormethoxy bedeutet; und
X², X³ Wasserstoff oder Fluor darstellen.

2. Verbindungen gemäss Anspruch 1 der Formel 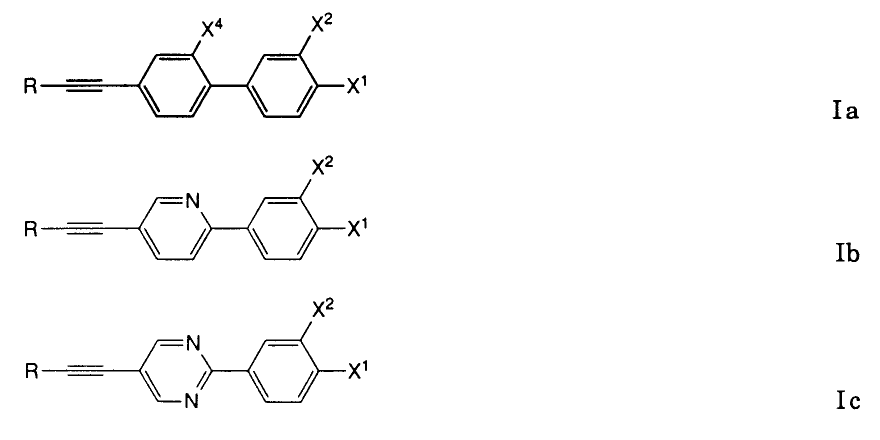 worin R eine Alkylgruppe mit 1 bis 12 Kohlenstoffatomen bedeutet; X⁴ Wasserstoff oder Fluor bedeutet, und X¹ und X² die in Anspruch 1 definierten Bedeutungen haben.

3. Verbindungen gemäss Anspruche 1 der allgemeinen Formeln 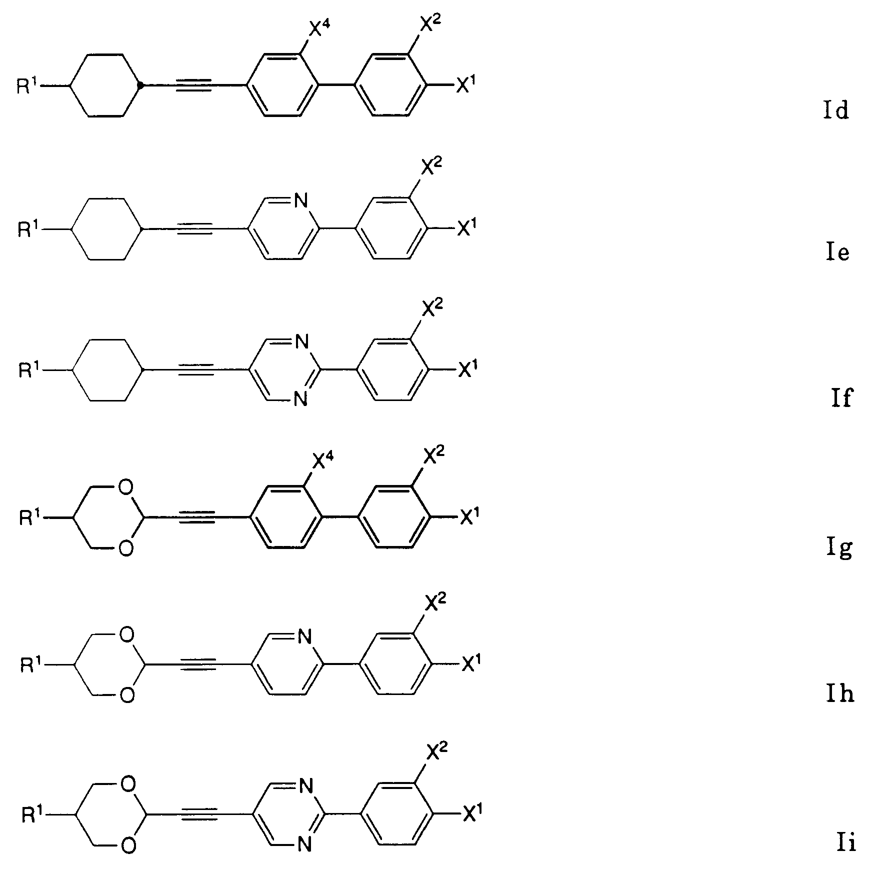 worin R¹, X¹ und X² wie in Anspruch 1 definiert sind, und X⁴ Wasserstoff oder Fluor bedeutet.

4. Verbindungen gemäss einem der Ansprüche 1 bis 3, dadurch gekennzeichnet, dass R Alkyl mit 1 bis 7 Kohlenstoffatomen bedeutet und R¹ Alkyl, Alkenyl oder Alkoxyalkyl mit 1 bis 7, bzw. 2 bis 7 Kohlenstoffatomen darstellt.

5. Verbindungen gemäss einem der Ansprüche 1, 2 oder 4, dadurch gekennzeichnet, dass R eine Alkyl Gruppe mit 2 bis 5 Kohlenstoffatomen bedeutet.

6. Verbindungen gemäss einem der Ansprüche 1, 3 oder 4, dadurch gekennzeichnet, dass R¹ Alkyl oder Alkenyl mit 2 bis 5 Kohlenstoffatome hat bedeutet, und A² trans-1,4-Cyclohexylen bedeutet.

7. Flüssigkristallines Gemisch, dadurch gekennzeichnet, dass mindestens eine Komponente eine Verbindung der in Anspruch 1 definierten Formel I ist.

8. Verwendung der Verbindungen der in Anspruch 1 definierten Formel I für optische oder elektro-optische Zwecke.

9. Verwendung flüssigkristalliner Gemische gemäss Anspruch 7 für optische oder elektro-optische Zwecke.

## Claims

1. Compounds of the general formula 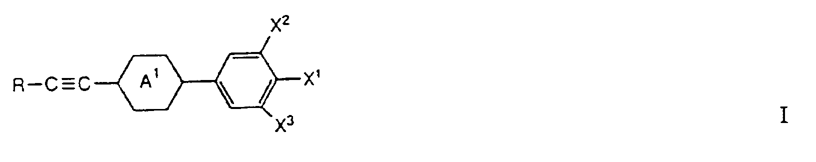 in which
R is an alkyl group having 1 to 12 carbon atoms or a group of the formula 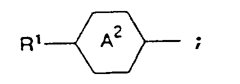
Ring A¹ is unsubstituted or fluorine-substituted 1,4-phenylene, pyridine-2,5-diyl or pyrimidine-2,5-diyl;
Ring A² is trans-1,4-cyclohexylene or trans-1,3-dioxane-2,5-diyl;
R¹ is an alkyl, alkenyl or alkoxyalkyl group having 1 to 12 or 2 to 12 carbon atoms respectively;
X¹ is fluorine, chlorine, bromine, trifluoromethyl, trifluoromethoxy or difluoromethoxy; and
X², X³ are hydrogen or fluorine.

2. Compounds according to Claim 1, of the formula 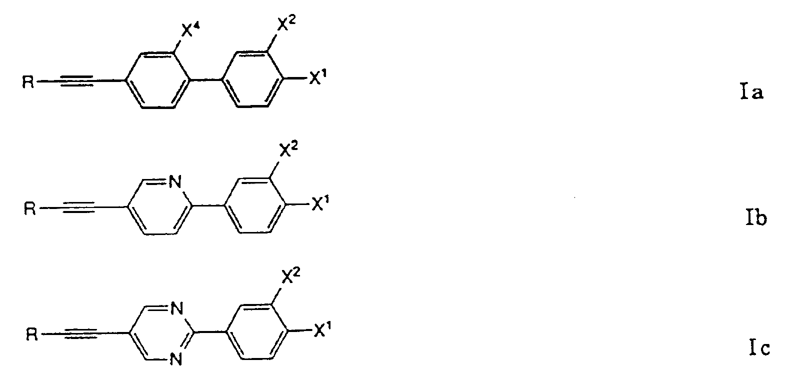 in which R is an alkyl group having 1 to 12 carbon atoms; X⁴ is hydrogen or fluorine, and X¹ and X² are as defined in Claim 1.

3. Compounds according to Claim 1, of the general formulae 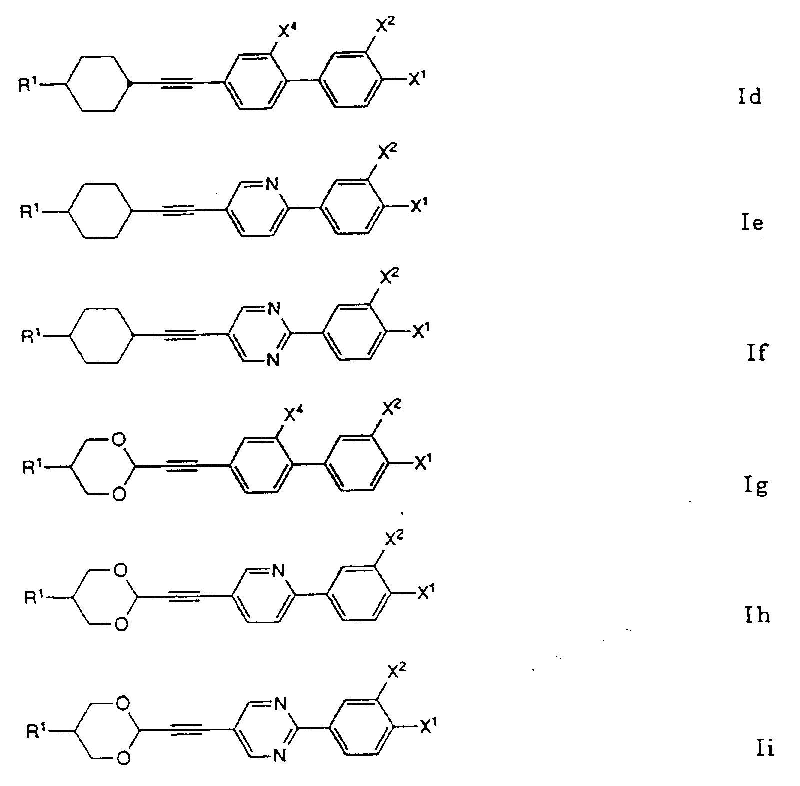 in which R¹, X¹ and X² are as defined in Claim 1, and X⁴ is hydrogen or fluorine.

4. Compounds according to one of Claims 1 to 3, characterized in that R is alkyl having 1 to 7 carbon atoms, and R¹ is alkyl, alkenyl or alkoxyalkyl having 1 to 7 or 2 to 7 carbon atoms respectively.

5. Compounds according to one of Claims 1, 2 and 4, characterized in that R is an alkyl group having 2 to 5 carbon atoms.

6. Compounds according to one of Claims 1, 3 and 4, characterized in that R¹ is alkyl or alkenyl having 2 to 5 carbon atoms, and A² is trans-1,4-cyclohexylene.

7. Liquid-crystalline mixture, characterized in that at least one component is a compound of the formula I defined in Claim 1.

8. Use of the compounds of the formula I defined in Claim 1 for optical or electro-optical purposes.

9. Use of liquid-crystalline mixtures according to Claim 7 for optical or electro-optical purposes.

## Revendications

1. Composés de formule générale 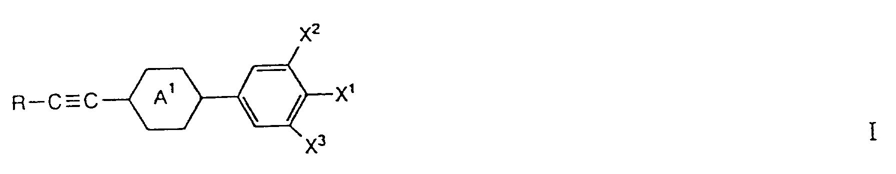 dans laquelle
R est un groupe alkyle ayant de 1 à 12 atomes de carbone ou un groupe de formule 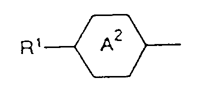
le noyau A¹ représente un radical 1,4-phénylène, pyridine-2,5-diyle ou pyrimidine-2,5-diyle, non substitué ou fluoré ;
le noyau A² est le radical trans-1,4-cyclohexylène ou trans-1,3-dioxanne-2,5-diyle ;
R¹ est un groupe alkyle, alcényle ou alcoxyalkyle ayant de 1 à 12 ou de 2 à 12 atomes de carbone ;
X¹ est le fluor, le chlore, le brome ou un radical trifluorométhyle, trifluorométhoxy ou difluorométhoxy ; et
X², X³ représentent l'hydrogène ou le fluor.

2. Composés selon la revendication 1, de formule 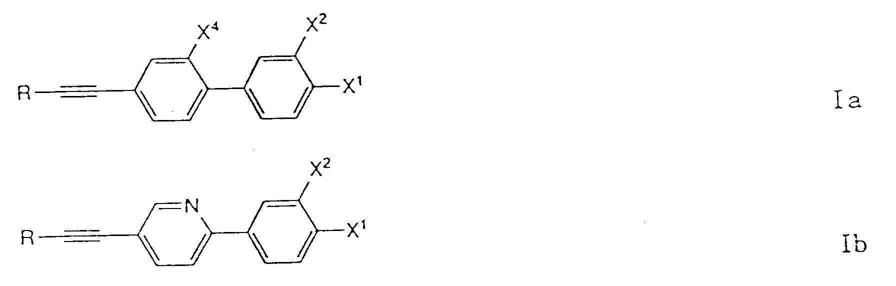 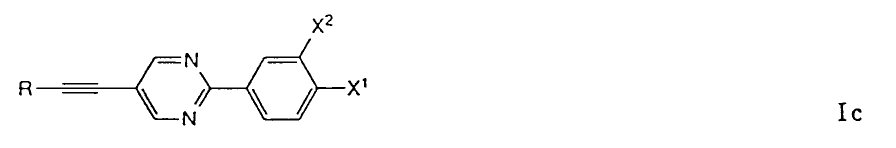 où R est un groupe alkyle ayant de 1 à 12 atomes de carbone ; X⁴ est un hydrogène ou un fluor, et X¹ et X² ont les significations données dans la revendication 1.

3. Composés selon la revendication 1, de formules générales 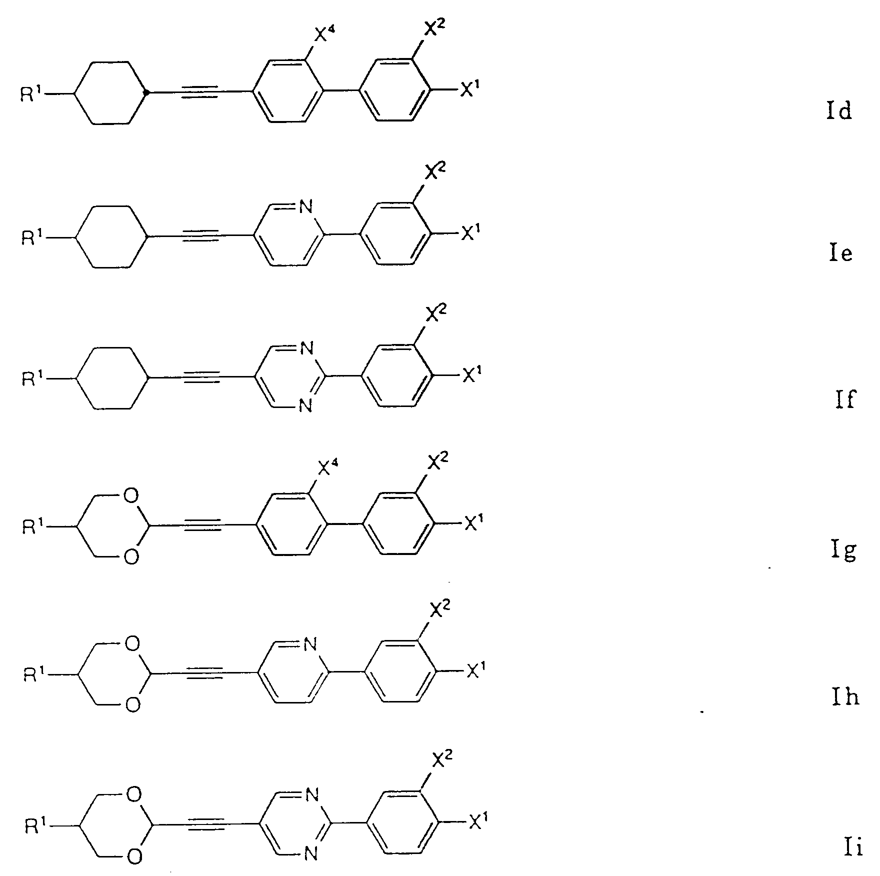 où R¹, X¹ et X² sont tels que définis dans la revendication 1, et X⁴ est un hydrogène ou un fluor.

4. Composés selon l'une des revendications 1 à 3, caractérisés en ce que R est un groupe alkyle ayant de 1 à 7 atomes de carbone, et R¹ est un groupe alkyle, alcényle ou alcoxyalkyle ayant de 1 à 7 ou de 2 à 7 atomes de carbone.

5. Composés selon l'une des revendications 1, 2 ou 4, caractérisés en ce que R est un groupe alkyle ayant de 2 à 5 atomes de carbone.

6. Composés selon l'une des revendications 1, 3 ou 4, caractérisés en ce que R¹ est un groupe alkyle ou alcényle ayant de 2 à 5 atomes de carbone, et A² est le radical trans-1,4-cyclohexylène.

7. Mélange mésomorphe, caractérisé en ce qu'au moins un constituant est un composé ayant la formule I définie dans la revendication 1.

8. Utilisation des composés ayant la formule I selon la revendication 1 pour des applications optiques ou électro-optiques.

9. Utilisation de mélanges mésomorphes selon la revendication 7 pour des applications optiques ou électro-optiques.
